# EUROPEAN PATENT SPECIFICATION

(11) **EP 2 081 615 B1**
(45) Date of publication and mention of the grant of the patent: **06.07.2011**
(21) Application number: 07842504.8
(22) Date of filing: 14.09.2007
(51) Int. Cl.: A61L 31/04, A61L 31/10, C08L 83/04

(54) **MEDICAL COMPONENTS HAVING COATED SURFACES EXHIBITING LOW FRICTION AND METHODS OF REDUCING STICKTION**
MEDIZINISCHE KOMPONENTEN MIT REIBUNGSARMEN BESCHICHTETEN OBERFLÄCHEN UND VERFAHREN ZUR REDUZIERUNG DER HAFTREIBUNG
COMPOSANTS MÉDICAUX À SURFACES REVÊTUES À FAIBLE FROTTEMENT ET PROCÉDÉS DE RÉDUCTION DE L'ADHÉRENCE

(30) Priority: 15.09.2006 US 844743 P
(43) Date of publication of application: 29.07.2009
(73) Proprietor: Becton, Dickinson and Company, Franklin Lakes, NJ 07417 (US)
(72) Inventor: WU, Shang-ren, Mahwah, New Jersey 07430 (US)
(74) Representative: von Kreisler Selting Werner
(86) International application number: PCT/US2007/078495
(87) International publication number: WO 2008/034060

(56) References cited:
- WO-A-99/47192
- WO-A-2004/064901
- WO-A-2004/083348
- US-A- 4 806 430
- US-A1- 2001 004 466

## Description

### BACKGROUND OF THE INVENTION

### Field of the Invention

This invention relates to medical components having surfaces in sliding engagement, such as syringe assemblies, coated with composition(s) comprising organopolysiloxane(s), methods to reduce static and kinetic friction between slidable surfaces, and articles of low friction prepared thereby.

### Description of Related Art

Certain devices require slow and controlled initiation and maintenance of sliding movement of one surface over another surface. It is well known that two stationary surfaces having a sliding relationship often exhibit sufficient resistance to initiation of movement that gradually increased force applied to one of the surfaces does not cause movement until a threshold force is reached, at which point a sudden sliding or shearing separation of the surfaces takes place. This sudden separation of stationary surfaces into a sliding relationship is herein referred to as "breakout" or "breakloose".

"Breakout force" refers to the force required to overcome static friction between surfaces of a syringe assembly that has been previously moved in a sliding relationship, but has been stationary ("parked" or not moved) for a short period of time (for example, milliseconds to hours). A less well known but important frictional force is "breakloose force", which refers to the force required to overcome static friction between surfaces of a syringe assembly that have not been previously moved in a sliding relationship or have been stationary for longer periods of time, often with chemical or material bonding or deformation of the surfaces due to age, sterilization, temperature cycling, or other processing.

Breakout and breakloose forces are particularly troublesome in liquid dispensing devices, such as syringes, used to deliver small, accurately measured quantities of a liquid by smooth incremental line to line advancement of one surface over a second surface. The problem is also encountered in devices using stopcocks, such as burets, pipets, addition funnels and the like where careful dropwise control of flow is desired.

The problems of excessive breakout and breakloose forces are related to friction. Friction is generally defined as the resisting force that arises when a surface of one substance slides, or tends to slide, over an adjoining surface of itself or another substance. Between surfaces of solids in contact, there may be two kinds of friction: (1) the resistance opposing the force required to start to move one surface over another, conventionally known as static friction, and (2) the resistance opposing the force required to move one surface over another at a variable, fixed, or predetermined speed, conventionally known as kinetic friction.

The force required to overcome static friction and induce breakout or breakloose is referred to as the "breakout force" or "breakloose force", respectively, and the force required to maintain steady slide of one surface over another after breakout or breakloose is referred to as the "sustaining force". Two main factors, sticktion and inertia, contribute to static friction and thus to the breakout or breakloose force. The term "stick" or "sticktion" as used herein denotes the tendency of two surfaces in stationary contact to develop a degree of adherence to each other. The term "inertia" is conventionally defined as the indisposition to motion which must be overcome to set a mass in motion. In the context of the present invention, inertia is understood to denote that component of the breakout or breakloose force which does not involve adherence.

Breakout or breakloose forces, in particular the degree of stick, vary according to the composition of the surfaces. In general, materials having elasticity show greater stick than non-elastic materials. The length of time that surfaces have been in stationary contact with each other also influences breakout and/or breakloose forces. In the syringe art, the term "parking" denotes storage time, shelf time, or the interval between filling and discharge. Parking time generally increases breakout or breakloose force, particularly if the syringe has been refrigerated or heated during parking.

A conventional approach to overcoming breakout or breakloose has been application of a lubricant to a surface to surface interface. Common lubricants used are hydrocarbon oils, such as mineral oils, peanut oil, vegetable oils and the like. Such products have the disadvantage of being soluble in a variety of fluids, such as vehicles commonly used to dispense medicaments. In addition, these lubricants are subject to air oxidation resulting in viscosity changes and objectionable color development. Further, they are particularly likely to migrate from the surface to surface interface. Such lubricant migration is generally thought to be responsible for the increase in breakout or breakloose force with time in parking.

Silicone oils are also commonly used as lubricants, are not subject to oxidation, but migration and stick do occur, and high breakout and/or breakloose forces are a problem. Polytetrafluoroethylene surfaces provide some seduction in breakout and/or breakloose forces, but this material is very expensive, and the approach has not been totally effective.

Thus there is a need for a better system to overcome high breakout and breakloose forces whereby smooth transition of two surfaces from stationary contact into sliding contact can be achieved.

### SUMMARY OF THE INVENTION

In some embodiments, the present invention provides a medical article comprising: (a) a chamber formed from a cyclic polyolefin and having an inner surface in sliding engagement with an exterior surface of a sealing member, wherein the inner surface of the chamber has a coating thereon prepared from a composition comprising a first organopolysiloxane having a viscosity ranging from 5,000·10⁻⁶ m²/s to 100,000·10⁻⁶ m²/s (5,000 centistokes to 100,000 centistokes), as measured with a Brookfield DV II + viscosimeter, the coating being adhered to the inner surface by crosslinking induced by irradiation with an isotope, electron beam or ultraviolet radiation; and (b) a sealing member having an exterior surface in sliding engagement with the interior surface of the chamber, the exterior surface of the sealing member having a coating thereon prepared from a composition comprising a second organopolysiloxane having a viscosity ranging from 10,000·10⁻⁶ m²/s to 500,000·10⁻⁶ m²/s (10,000 centistokes to 500,000 centistokes), the coating being adhered to the exterior surface by crosslinking induced by irradiation with an isotope, electron beam, or ultraviolet radiation,
wherein the viscosity of the organopolysiloxane coating the chamber is less than the viscosity of the organopolysiloxane coating the sealing member.

In other embodiments, the present invention provides a method for lubricating the interface between an inner surface of a chamber formed from a cyclic polyolefin and an exterior surface of a sealing member of a medical article, comprising the steps of: (a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes) as measured with a Brookfield DV II + viscosimeter; (b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes; and (c) irradiating the coating of the inner surface of the chamber and the coating of the exterior surface of the sealing member with an isotope, electron beam, or ultraviolet radiation.

In other embodiments, the present invention provides a method for reducing breakloose force between an inner surface of a chamber formed from a cyclic polyolefin and an exterior surface of a sealing member of a medical article, comprising the steps of (a) applying a coating onto an inner surface of the chamber, the coating being prepared from a composition comprising a first oranopolysiloxane having a viscosity ranging from 5,000·10⁻⁶ m²/s to 100,000·10⁻⁶ m²/s (5,000 centistokes to 100,000 centistokes) as measured with a Brookfield DV II + viscosimeter; (b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes) wherein the viscosity of the organopolysiloxane coating the chamber is less than the viscosity of the organopolysiloxane coating the sealing member, and (c) irradiating the coating of the inner surface of the chamber and the coating of the exterior surface of the sealing member with an isotope, electron beam, or ultraviolet radiation.

In other embodiments, the present invention provides a method for reducing sustaining force between an inner surface of a chamber formed from a cyclic polyolefin and an exterior surface of a sealing member of a medical article, comprising the steps of: (a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes as measured with a Brookfield DV II + viscometer; (b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes wherein the viscosity of the organopolysiloxane coating the chamber is less than the viscosity of the organopolysiloxane coating the sealing member; and (c) irradiating the coating of the inner surface of the chamber and the coating of the exterior surface of the sealing member with an isotope, electron beam, or ultraviolet radiation.

In other embodiments, the present invention provides a method for reducing sticktion between an inner surface of a chamber formed from a cyclic polyolefin and an exterior surface of a sealing member of a medical article, comprising the steps of: (a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes as measured with a Brookfield DV II + viscometer; (b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes) wherein the viscosity of the organopolysiloxane coating the chamber is less than the viscosity of the organopolysiloxane coating the sealing member; and (c) irradiating the coating of the inner surface of the chamber and the coating of the exterior surface of the sealing member with an isotope, electron beam, or ultraviolet radiation.

### BRIEF DESCRIPTION OF THE DRAWINGS

The present invention will best be understood from the following description of specific embodiments when read in connection with the accompanying drawings:

Fig. 1 is a graph of infusion pump actuation force test results of Comparative Sample Group 1 at a feed rate of 0.1 ml/hr for a syringe assembly having a syringe barrel and stopper not treated with gamma radiation;

Fig. 2 is a graph of infusion pump actuation force test results of Comparative Sample Group 1 at a feed rate of 1.0 ml/hr for a syringe assembly having a syringe barrel and stopper not treated with gamma radiation;

Fig. 3 is a graph of infusion pump actuation force test results of Comparative Sample Group 1 at a feed rate of 10 ml/hr for a syringe assembly having a syringe barrel and stopper not treated with gamma radiation;

Fig. 4 is a graph of infusion pump actuation force test results of Sample Group 3 at a feed rate of 0.1 ml/hr for a syringe assembly having a syringe barrel and stopper treated with gamma radiation according to the present invention;

Fig. 5 is a graph of infusion pump actuation force test results of Sample Group 3 at a feed rate of 1.0 ml/hr for a syringe assembly having a syringe barrel and stopper treated with gamma radiation according to the present invention;

Fig. 6 is a graph of infusion pump actuation force test results of Sample Group 3 at a feed rate of 10 ml/hr for a syringe assembly having a syringe barrel and stopper treated with gamma radiation according to the present invention;

Fig. 7 is a graph of infusion pump actuation force test results of Comparative Sample Group 4 at a feed rate of 0.1 ml/hr for a syringe assembly having a syringe barrel and stopper not treated with gamma radiation;

Fig. 8 is a graph of infusion pump actuation force test results of Comparative Sample Group 4 at a feed rate of 1.0 ml/hr for a syringe assembly having a syringe barrel and stopper not treated with gamma radiation;

Fig. 9 is a graph of infusion pump actuation force test results of Comparative Sample Group 4 at a feed rate of 10 ml/hr for a syringe assembly having a syringe barrel and stopper not treated with gamma radiation;

Fig. 10 is a graph of infusion pump actuation force test results of Sample Group 5 at a feed rate of 0.1 ml/hr for a syringe assembly having a syringe barrel and stopper treated with gamma radiation according to the present invention;

Fig. 11 is a graph of infusion pump actuation force test results of Sample Group 5 at a feed rate of 1.0 ml/hr for a syringe assembly having a syringe barrel and stopper treated with gamma radiation according to the present invention;

Fig. 12 is a graph of infusion pump actuation force test results of Sample Group 5 at a feed rate of 10 ml/hr for a syringe assembly having a syringe barrel and stopper treated with gamma radiation according to the present invention;

Fig. 13 is a graph of infusion pump actuation force test results of Sample Group 6 at a feed rate of 0.1 ml/hr for a syringe assembly having a syringe barrel and stopper treated with gamma radiation according to the present invention;

Fig. 14 is a graph of infusion pump actuation force test results of Sample Group 6 at a feed rate of 1.0 ml/hr for a syringe assembly having a syringe barrel and stopper treated with gamma radiation according to the present invention;

Fig. 15 is a graph of infusion pump actuation force test results of Sample Group 6 at a feed rate of 10 ml/hr for a syringe assembly having a syringe barrel and stopper treated with gamma radiation according to the present invention;

Fig. 16 is a graph of infusion pump actuation force test results of Sample Group 10 at a feed rate of 0.1 ml/hr for a syringe assembly having a syringe barrel and stopper treated with gamma radiation according to the present invention;

Fig. 17 is a graph of infusion pump actuation force test results of Sample Group 10 at a feed rate of 1.0 ml/hr for a syringe assembly having a syringe barrel and stopper treated with gamma radiation according to the present invention; and

Fig. 18 is a graph of infusion pump actuation force test results of Sample Group 10 at a feed rate of 10 ml/hr for a syringe assembly having a syringe barrel and stopper treated with gamma radiation according to the present invention.

### DETAILED DESCRIPTION OF THE INVENTION

Other than in the operating examples, or where otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements. Furthermore, when numerical ranges of varying scope are set forth herein, it is contemplated that any combination of these values inclusive of the recited values may be used.

Also, it should be understood that any numerical range recited herein is intended to include all sub-ranges subsumed therein. For example, a range of "1 to 10" is intended to include all sub-ranges between and including the recited minimum value of 1 and the recited maximum value of 10, that is, having a minimum value equal to or greater than 1 and a maximum value of equal to or less than 10.

The present invention provides a medical article having a chamber formed from a cyclic polyolefin, the chamber having an inner surface in sliding and/or sealing frictional engagement with an exterior surface of a sealing member of the article. The inner surface of the chamber and exterior surface of the sealing member have certain organopolysiloxane coatings adhered to the respective surfaces by crosslinking induced by irradiation with an isotope, electron beam, or ultraviolet radiation.

The respective surfaces of the inner surface of the chamber and the exterior surface of the sealing member can be in frictional engagement. When used in a medical article, the effects of the present invention can reduce the force required to achieve breakout, breakloose and/or sustaining forces, whereby transition of surfaces from stationary contact to sliding contact occurs without a sudden surge. When breakout or breakloose is complete and the surfaces are in sliding contact, they slide smoothly upon application of very low sustaining force. The effect achieved by the methods of the present invention can be of long duration, and articles, such as syringes, can retain the advantages of low breakout, low breakloose and low sustaining forces for several years. When the chamber is part of a liquid dispensing device, small highly accurate increments of liquid may be dispensed repeatedly without sudden surges. Thus, a syringe including a chamber treated according to the present invention can be used to administer a medicament to a patient without the danger of surges whereby accurate control of dosage and greatly enhanced patient safety are realized.

As used herein, "medical article" means an article or device that can be useful for medical treatment. Non-limiting examples of medical articles include syringe assemblies, drug cartridges, needleless injectors, liquid dispensing devices and liquid metering devices. In some embodiments, the medical article is a syringe assembly comprising a syringe chamber or barrel (for receiving water, saline or a medicament, for example) and a sealing member.

The chamber is formed from a cyclic polyolefin, non-limiting examples of which include norbornene polymers such as are disclosed in U.S. Patents Nos. 6,525,144, 6,511,756, 5,599,882, and 5,034,482 (each of Nippon Zeon), 7,037,993, 6,995,226, 6,908,970, 6,653,424 and 6,486,264 (each of Zeon Corp.), 7,026,401, and 6,951,898 (Ticona), 6,063,886 (Mitsui Chemicals), 5,866,662, 5,856,414, 5,623,039 and 5,610,253 (Hoechst), 5,854,349, and 5,650,471 (Mitsui Petrochemical and Hoechst) and as described in "Polycyclic olefins", process Economics Program (July 1998) SRI Consulting. Non-limiting examples of suitable cyclic polyolefins include Apel™ cyclic polyolefins available from Mitsui Petrochemical, Topas™ cyclic polyolefins available from Ticona Engineering Polymers, Zeonor™ or Zeonex™ cyclic polyolefins available from Zeon Corporation, and cyclic polyolefins available from Promerus LLC.

The polyolefin can contain a small amount, generally from about 0.1 to 10 percent, of an additional polymer incorporated into the composition by copolymerization with the appropriate monomer. Such copolymers may be added to the composition to enhance other characteristics of the final composition, and may be, for example, polyacrylate, polystyrene and the like.

In some embodiments, the chamber may be constructed of a polyolefin composition which includes a radiation stabilizing additive to impart radiation stability to the container, such as a mobilizing additive which contributes to the radiation stability of the container, such as for example those disclosed in U.S. Patent Nos. 4,959,402 and 4,994,552, assigned to Becton, Dickinson and Company.

The other component of the medical article in contact with the chamber is the sealing member. The sealing member can be formed from any elastomeric or plastic material. Elastomers are used in many important and critical applications in medical devices and pharmaceutical packaging. As a class of materials, their unique characteristics, such as flexibility, resilience, extendability, and sealability, have proven particularly well suited for products such as catheters, syringe tips, drug vial articles, tubing, gloves and hoses. Three primary synthetic thermoset elastomers typically are used in medical applications: polyisoprene rubber, silicone rubber, and butyl rubber. Of the three rubbers, butyl rubber has been the most common choice for articles due to its high cleanness and permeation resistance which enables the rubber to protect oxygen- and water-sensitive drugs.

Suitable butyl rubbers useful in the method of the present invention include copolymers of isobutylene (about 97-98%) and isoprene (about 2-3%). The butyl rubber can be halogenated with chlorine or bromine. Suitable butyl rubber vulcanizates can provide good abrasion resistance, excellent impermeability to gases, a high dielectric constant, excellent resistance to aging and sunlight, and superior shock-absorbing and vibration-damping qualities to articles formed therefrom. Non-limiting examples of suitable rubber stoppers include those available from West Pharmaceuticals, American Gasket Rubber, Stelmi and Helvoet Rubber & Plastic Technologies BV.

Other useful elastomeric copolymers include, without limitation, thermoplastic elastomers, thermoplastic vulcanizates, styrene copolymers such as styrene-butadiene (SBR or SBS) copolymers, styrene-isoprene (SIS) block polymers or styrene-isoprene/butadiene (SIBS), in which the content of styrene in the styrene block copolymer ranges from about 10% to about 70%, and preferably from about 20% to about 50%. Non-limiting examples of suitable styrene-butadiene stoppers are available from Firestone Polymers, Dow, Reichhold, Kokoku Rubber Inc. and Chemix Ltd. Other suitable thermoplastic elastomers are available from GLS, Tecknor Apex, AES, Mitsubishi and Solvay Engineered Polymers, for example. The elastomer composition can include, without limitation, antioxidants and/or inorganic reinforcing agents to preserve the stability of the elastomer composition.

In some embodiments, the sealing member can be a stopper, O-ring, plunger tip or piston, for example. Syringe plunger tips or pistons typically are made of a compressible, resilient material such as rubber, because of the rubber's ability to provide a seal between the plunger and interior housing of the syringe. Syringe plungers, like other equipment used in the care and treatment of patients, have to meet high performance standards, such as the ability to provide a tight seal between the plunger and the barrel of the syringe.

The coating is applied to at least a portion of at least one surface of the chamber to be placed in frictional engagement with an opposed surface of another component The opposed surface of the other component of the medical device, such as the sealing member, is coated with another coating as described below. Methods for coating the surfaces are discussed in detail below.

The chamber is coated with a coating prepared from a composition comprising one or more organopolysiloxane(s) having a viscosity ranging from about 5,000 centistokes to about 100,000 centistokes, prior to any curing step. In some embodiments, the organopolysiloxane has a viscosity ranging from 5,000·10⁻⁶ m²/s to 15,000·10⁻⁶ m²/s (5,000 centistokes to 15,000 centistokes (cst)). In other embodiments, the organopolysiloxane has a viscosity of 12,500·10⁻⁶ m²/s (12,500 centistokes). The viscosity can be measured using a Brookfield DV II+ viscometer.

The sealing member is coated with one or more organopolysiloxanes, such as are discussed above, having a viscosity of 10,000·10⁻⁶ m²/s to 500,000·10⁻⁶ m²/s (10,000 to 500,000 cst). In some embodiments, the sealing member is coated with polydimethylsiloxane having a viscosity of 10,000·10⁻⁶ m²/s to 500,000·10⁻⁶ m/s (10,000 to 300,000 cst), prior to any curing step. For example, the viscosity of the organopolysiloxane on the sealing member can be 12,500·10⁻⁶, 100,000·10⁻⁶ or 300,000·10⁻⁶ m²/s (12,500, 100,000 or 300,000 cst).

The viscosity of the organopolysiloxane coating the chamber can be greater than the viscosity of the organopolysiloxane coating the sealing member. In other embodiments, the viscosity of the organopolysiloxane coating the chamber can be less than or equal to the viscosity of the organopolysiloxane coating the sealing member. For example, the viscosity of the organopolysiloxane coating the chamber can be 12,500·10 m²/s (12,500 cst), while the viscosity of the organopolysiloxane coating the sealing member can be 12,500·10⁻⁶, 100,000·10⁻⁶ or 300,000·10⁻⁶ m²/s (12,500, 100,000 or 300,000 cst). The organopolysiloxane coating the chamber can be the same or different from the organopolysiloxane coating the sealing member, for example the number or type of atoms in the organopolysiloxane can be different, the viscosity or the molecular weight can be different.

In some embodiments, the organopolysiloxane comprises an alkyl-substituted organopolysiloxane, for example as is represented by the following structural formula (I): 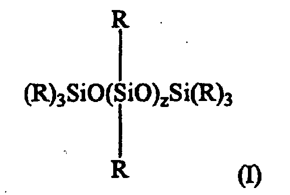 wherein R is alkyl and Z is about 30 to about 4,500. In some embodiments, the organopolysiloxane of Formula (I) can be represented by the following structural formula (II): 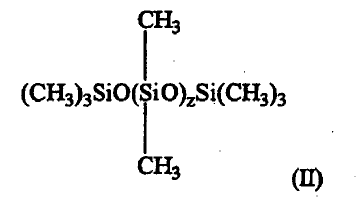 wherein Z can be as above, or for example, can be about 300 to about 2,000; about 300 to about 1,800; or about 300 to about 1,350. In some embodiments, the organopolysiloxane is a polydimethylsiloxane, such as DOW CORNING® 360 polydimethylsiloxane or NUSIL polydimethylsiloxane having a viscosity ranging from 100·10⁻⁶ to 1,000,000·10⁻⁶ m²/s (100 to 1,000,000 cst).

In other embodiments, the organopolysiloxane comprises one or more curable or reactive functional groups, such as alkenyl groups. As used herein, the term "cure" as used in connection with a composition, i.e., a "cured composition" or a "cured coating" shall mean that at least a portion of the crosslinkable components which form the composition are at least partially crosslinked. As used herein, the term "curable", as used in connection with a component of the composition, means that the component has functional groups capable of being crosslinked, for example, alkenyl groups such as vinyl groups. In certain embodiments of the present invention, the crosslink density of the crosslinkable components, i.e., the degree of crosslinking, ranges from 5% to 100% of complete crosslinking. One skilled in the art will understand that the presence and degree of crosslinking, i.e., the crosslink density, can be determined by a variety of methods, such as dynamic mechanical thermal analysis (DMTA). This method determines the glass transition temperature and crosslink density of free films of coatings or polymers. These physical properties of a cured material are related to the structure of the crosslinked network.

In some embodiments, the organopolysiloxane comprises at least one alkenyl group. Each alkenyl group can be independently selected from the group consisting of vinyl, allyl, propenyl, butenyl, pentenyl, hexenyl, heptenyl, octenyl, nonenyl, and decenyl. One skilled in the art would understand that the organopolysiloxane can comprise one or more of any of the above types of alkenyl groups and mixtures thereof. In some embodiments, at least one alkenyl group is vinyl. Higher alkenyl or vinyl content provides more efficient crosslinking.

In some embodiments, the organopolysiloxane can be represented by the following structural formulae (III) or (IV): 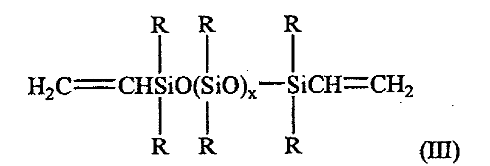 or 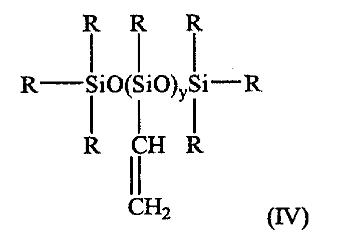 wherein R is alkyl, haloalkyl, aryl, haloaryl, cycloalkyl, silacyclopentyl, aralkyl, and mixtures thereof; X is about 60 to about 1000, preferably about 200 to about 320; and y is about 3 to about 25. Copolymers and mixtures of these polymers are also contemplated.

Non-limiting examples of useful vinyl functional organopolysiloxanes include: vinyldimethylsiloxy terminated polydimethylsiloxanes; trimethylsiloxy terminated vinylmethyl, dimethylpolysiloxane copolymers; vinyldimethylsiloxy terminated vinylmethyl, dimethylpolysiloxane copolymers; divinylmethylsiloxy terminated polydimethylsiloxanes; vinyl, n-butylmethyl terminated polydimethylsiloxanes; and vinylphenylmethylsiloxy terminated polydimethylsiloxanes.

In some embodiments, a mixture of siloxane polymers selected from those of Formulae II, III and/or IV can be used. For example, the mixture can comprise two different molecular weight vinyldimethylsiloxy terminated polydimethylsiloxane polymers, wherein one of the polymers has an average molecular weight of about 1,000 to about 25,000 and preferably about 16,000, and the other polymer has an average molecular weight of about 30,000 to about 71,000 and preferably about 38,000. Generally, the lower molecular weight siloxane can be present in amounts of about 20% to about 80%, such as about 60% by weight of this mixture; and the higher molecular weight siloxane can be present in amounts of about 80% to about 20%, such as about 40% by weight of this mixture.

Another non-limiting example of a suitable vinyl functional organopolysiloxane is (7.0-8.0% vinylinethylsiloxane) - dimethylsiloxane copolymer, trimethylsiloxy terminated, such as VDT-731 vinylmethylsiloxane copolymer which is commercially available from Gelest, Inc. of Morrisville, PA.

In some embodiments, the organopolysiloxane can comprise at least two polar groups. Each polar group can be independently selected from the group consisting of acrylate, methacrylate, amino, imino, hydroxy, epoxy, ester, alkyloxy, isocyanate, phenolic, polyurethane oligomeric, polyamide oligomeric, polyester oligomeric, polyether oligomeric, polyol, and carboxypropyl groups. One skilled in the art would understand that the organopolysiloxane can comprise one or more of any of the above polar groups and mixtures thereof. Preferably, these organopolysiloxanes are not moisture-curable.

In some embodiments, the polar groups are acrylate groups, for example acryloxypropyl groups. In other embodiments, the polar groups are methacrylate groups, such as methacryloxypropyl groups.

The organopolysiloxane having polar groups can further comprise one or more alkyl groups and/or aryl groups, such as methyl groups, ethyl groups, or phenyl groups.

Non-limiting examples of such organopolysiloxanes include [15-20% (acryloxypropyl)methylsiloxane] - dimethylsiloxane copolymer, such as UMS-182 acrylate functional siloxane, which is available from Gelest, Inc. of Morrisville, PA, and SILCOLEASE® PC970 acrylated silicone polymer, which is available from Rhodia-Silicones.

In other embodiments, such an organopolysiloxane can be represented by the formula (V): 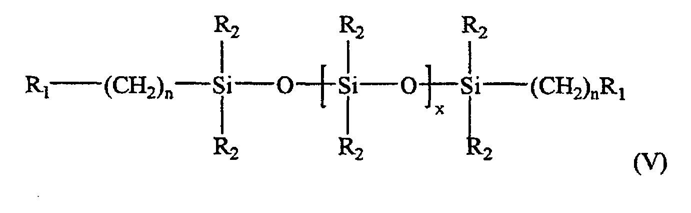 wherein R₁ is selected from the group consisting of acrylate, methacrylate, amino, imino, hydroxy, epoxy, ester, alkyloxy, isocyanate, phenolic, polyurethane oligomeric, polyamide oligomeric, polyester oligomeric, polyether oligomeric, polyol, carboxypropyl, and fluoro groups; and R₂ is alkyl, n ranges from 2 to 4, and x is an integer sufficient to give the lubricant a viscosity of 5,000·10⁻⁶ to 100,000·10⁻⁶ m²/s (5,000 to 100,000 cst).

While not wishing to be bound by any theory, it is believed that the polar siloxanes may help reduce the coefficient of friction between the engaged surfaces. Also, after irradiation, it is believed that the viscosity of the polar siloxane may increase and improve the binding of the coating to substrate.

In some embodiments, the organopolysiloxane can further comprise one or more fluoro groups, such as -F or fluoroalkyl groups such as trifluoromethyl groups. Other useful organopolysiloxanes include polyfluoroalkylmethyl siloxanes and fluoroalkyl, dimethyl siloxane copolymers.

In some embodiments, the composition can further comprise one or more cyclic siloxane(s), for example octamethylcyclotetrasiloxane and/or decamethylcyclopentasiloxane.

In some embodiments, the organopolysiloxane can be represented by the following structural formula (VI): 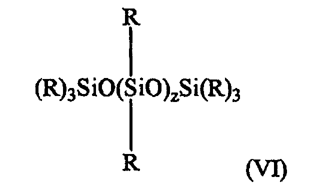 wherein R is haloalkyl, aryl (such as phenyl), haloaryl, cycloalkyl, silacyclopentyl, aralkyl, and mixtures thereof; and Z is about 20 to about 1,800.

In some embodiments, the organopolysiloxane comprises at least two pendant hydrogen groups. Non-limiting examples of suitable organopolysiloxanes comprising at least two pendant hydrogen groups include organopolysiloxanes having pendant hydrogen groups along the polymer backbone or terminal hydrogen groups. In some embodiments, the organopolysiloxane can be represented by the following structural formulae (VII): 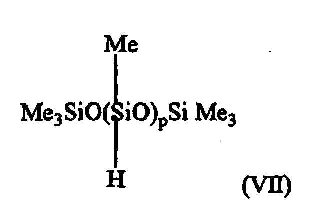 wherein p is about 8 to about 125, for example about 30. In other embodiments, the organopolysiloxane can be represented by the following structural formula (VIII):

HMe₂SiO(Me₂SiO)ₚSiMe₂H (VIII)

wherein p is about 140 to about 170, for example about 150 to about 160. A mixture of these polymers can be used comprising two different molecular weight materials. For example, about 2% to about 5% by weight of the mixture of a trimethylsiloxy terminated polymethylhydrosiloxane having an average molecular weight of about 400 to about 7,500, for example about 1900, can be used in admixture with about 98% to about 95% of a dimethylhydro siloxy-terminated polymethylhydrogensiloxane having an average molecular weight of about 400 to about 37,000 and preferably about 12,000. Non-limiting examples of useful organopolysiloxanes comprising at least two pendant hydrogen groups include dimethylhydro terminated polydimethylsiloxanes; methylhydro, dimethylpolysiloxane copolymers; dimethylhydrosiloxy terminated methyloctyl dimethylpolysiloxane copolymers; and methylhydro, phenylmethyl siloxane copolymers.

In some embodiments, the composition comprises hydroxy functional siloxanes, for example a hydroxy functional siloxane comprising at least two hydroxyl groups, such as for example: 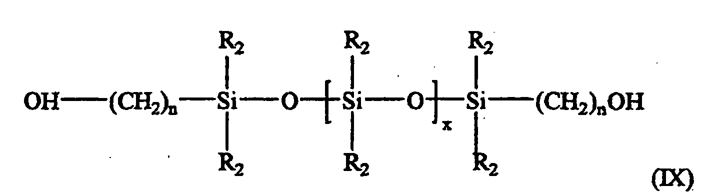 wherein R₂ is alkyl, n ranges from Q to 4, and x is an integer sufficient to give the lubricant a viscosity of 5,000·10⁻⁶ to 100,000·10⁻⁶ m² m²/s (5,000 to 100,000 cst). In some embodiments, moisture-curable siloxanes which have moisture-curing character as a result of functionality include siloxanes having functional groups such as: alkoxy, aryloxy; oxime; epoxy; -OOCR, N,N-dialkylamino; N,N-dialkylaminoxy; N-alkylamido; -O-NH-C(O)-R ; -O-C(=NCH₃)-NH-CH₃, -O-C(CH3)=CH2; wherein R is H or hydrocarbyl. As used herein, "moisture-curable" means that the siloxane is curable at ambient conditions in the presence of atmospheric moisture.

Mixtures of one or more of the organopolysiloxanes discussed above can be used in the present invention.

In some embodiments, the organopolysiloxane comprises about 90 to about 100 weight percent of the composition. In other embodiments, the organopolysiloxane comprises about 95 to about 100 weight percent of the composition. In other embodiments, the organopolysiloxane comprises 100 weight percent of the composition.

In some embodiments, the composition further comprises a catalytic amount of a catalyst for promoting crosslinking of crosslinkable groups of the organopolysiloxane(s). Non-limiting examples of suitable catalysts for promoting ultraviolet radiation cure include any suitable photoinitiator which is capable of initiating polymerization of the reactive silicone polymer upon exposure to UV light. Non-limiting examples of useful UV light-induced polymerization photoinitiators include ketones such as benzyl and benzoin, and acyloins and acyloin ethers, such as alpha-hydroxy ketones. Non-limiting examples of available products include IRGACURE 184 (1-hydroxycyclohexyl phenyl ketone), IRGACURE 907 (2-methyl-1-[4-(methylthio)phenyl]-2-(4-morpholinyl)-1-propanone), IRGACURE 369 (2-benzyl-2-N,N-dimethylamino-1-(4-morpholinophenyl)-1-butanone), IRGACURE 500 (the combination of 50% 1-hydroxy cyclohexyl phenyl ketone and 50% benzophenone), IRGACURE 651 (2,2-dimethoxy-1,2-diphenylethan-1-one), IRGACURE 1700 (the combination of 25% bis(2,6-dimethoxybenzoyl-2,4-, 4-trimethyl pentyl) phosphine oxide and 75% 2-hydroxy-2-methyl-1-phenyl-propan-1-one), DAROCUR 1173 (2-hydroxy-2-methyl-1-phenyl-propan-1-one), and DAROCUR 4265 (the combination of 50% 2,4,6-trimethylbenzoyldiphenyl-phosphine oxide and 50% 2-hydroxy-2-methyl-1-phenyl-propan-1-one), all of which are available from CIBA Corp., Tarrytown, N.Y.; and SARCURE SR-1121 (2-hydroxy-2-methyl-1-phenyl propanone) and ESACURE KIP-100F (a mixture of polymeric photoinitiators in 2-hydroxy-2-methyl-1-phenyl-propan-1-one), both of which are commercially available from Sartomer, Inc. of Exton, Pa. Of course, mixtures of different photoinitiators may also be used. The photoinitiator is desirably in a liquid form to ensure appropriate mixing and distribution within the composition, although solid photoinitiators may also be used, provided that they are soluble in organopolysiloxane to provide the composition as a homogeneous fluid. The photoinitiator should be present in an amount sufficient to provide the desired rate of photopolymerization, dependent in part on the light source and the extinction coefficient of the photoinitiator. Typically, the photoinitiator components will be present at a total weight of about 0.01 to about 10%, more preferably from about 0.1 to about 5%, based on the total weight of the composition.

The components of the composition can be formulated in a single composition or two compositions that are mixed prior to application, for example, to separate a catalyst from crosslinkable components until shortly before application. A non-limiting example of a suitable two component composition is a two-part LSR silicone composition commercially available from GE Silicones.

Application of a coating to the inner surface of the chamber or outer surface of the sealing member may be accomplished by any suitable method, as, for example, dipping, brushing, spraying and the like. The composition may be applied neat or it may be applied in a solvent, such as low molecular weight silicone, non-toxic chlorinated or fluorinated hydrocarbons, for example, 1,1,2-trichloro-1,2,2-trifluoroethane, freon or conventional hydrocarbon solvents such as alkanes, toluene, petroleum ether and the like where toxicology is not considered important. The solvent is subsequently removed by evaporation. The coating may be of any convenient thickness and, in practice, the thickness will be determined by such factors as the quantity applied, viscosity of the lubricant and the temperature of application. For reasons of economy, the coating preferably is applied as thinly as practical, since no significant advantage is gained by thicker coatings. The exact thickness of the coating does not appear to be critical and very thin coatings, i.e., one or two microns exhibit effective lubricating properties. While not necessary for operability, it is desirable that the thickness of the coating be substantially uniform throughout.

The coating can be partially or fully crosslinked after application or partially crosslinked to attach to the substrate, and then fully crosslinked at a later time.

The coated chamber and coated sealing member are subjected to irradiation with an isotope (such as gamma radiation), electron beam or ultraviolet radiation. It is believed that the radiation treatment induces cross-linking in the organopolysiloxane, whereby the organopolysiloxane is converted to a high molecular weight three dimensional polymer network. It is further believed that the radiation treatment can also induce crosslinking of the organopolysiloxane to the surface(s).

This technique has the advantage of sterilizing as well, which is useful in medical applications. Radiation sterilization in the form of ionizing radiation commonly is used in hospitals for medical devices such as catheters, surgical items, and critical care tools. Gamma irradiation is the most popular form of radiation sterilization and typically is used when materials are sensitive to the high temperature of autoclaving but are compatible with ionizing radiation. Gamma irradiation exerts a microbicidal effect by oxidizing biological tissue, and thus provides a simple, rapid and efficacious method of sterilization. Gamma rays are used either from a cobalt-60 (⁶⁰Co) isotope source or from a machine-generated accelerated electron source. Sufficient exposures are achieved when the materials to be sterilized are moved around an exposed ⁶⁰Co source for a defined period of time. The most commonly used validated dose for sterilizing medical articles is about 10 to about 100 kGy, such as for example, 25 to 50 kGy. In some embodiments, it is preferred that the chamber and/or the sealing member is treated with at least 25 kGy of radiation.

In some embodiments, a surface lubricant layer about 0.3 to 10, preferably about 0.8 to 4.0 microns thick may be applied over the organopolysiloxane coating before or after curing. The surface lubricant can be conventional silicone oil (organopolysiloxane) of viscosity 100·10⁻⁶ to 60,000·10⁻⁶ (100 to 60,00), preferably 1000·10⁻⁶ to 12,500·10⁻⁶ m²/s (1000 to 12,500 cst). The surface lubricating layer may be applied by any of the conventional methods described above. The preferred methods for applying the surface lubricant are by spraying or dipping the syringe barrel into a solution, about 4% by weight, of the surface lubricant in a solvent such as chloroform, dichloromethane or preferably a chlorofluorocarbon, such as FREON™ TF. The surface lubricant may optionally be lightly crosslinked by plasma treatment.

In some embodiments, the coated articles are subjected to a sterilization treatment. Many sterilization techniques are available today to sterilize medical devices to eliminate living organisms such as bacteria, yeasts, mold and viruses. Commonly used sterilization techniques used for medical devices include autoclaving, ethylene oxide (EtO) or gamma irradiation, as well as more recently introduced systems that involve low-temperature gas plasma and vapor phase sterilants.

One common sterilization technique is steam sterilization or autoclaving, which is a relatively simple process that exposes an article, for example, to saturated steam at temperatures of over 120°C for a minimum of twenty minutes at a pressure of about 120 kPa. The process is usually carried out in a pressure vessel designed to withstand the elevated temperature and pressure to kill microorganisms by destroying metabolic and structural components essential to their replication. Autoclaving is the method of choice for sterilization of heat-resistant surgical equipment and intravenous fluid as it is an efficient, reliable, rapid, relatively simple process that does not result in toxic residues.

Thus, in some embodiments, the present invention provides a method for lubricating the interface between an inner surface of a chamber formed from a cyclic polyolefin and an exterior surface of a sealing member of a medical article, comprising the steps of: (a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes); (b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes); and (c) irradiating the coating of the inner surface of the chamber and the coating of the exterior surface of the sealing member with an isotope, electron beam, or ultraviolet radiation.

In other embodiments, the present invention provides a method for reducing breakloose force between an inner surface of a chamber formed from a cyclic polyolefin and an exterior surface of a sealing member of a medical article, comprising the steps of (a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes); (b) applying a coating onto an exterior surface of the sealing member, the coating being prepared from a composition comprising a second organopolysiloxane having a viscosity ranging from 10,000·10 m²/s to 500,000·10⁻⁶ m²/s (10,000 centistokes to 500,000 centistokes); and (c) irradiating the coating of the inner surface of the chamber and the coating of the exterior surface of the sealing member with an isotope, electron beam or ultraviolet radiation.

In other embodiments, the present invention provides a method for reducing sustaining force between an inner surface of a chamber formed from a cyclic polyolefin and an exterior surface of a sealing member of a medical article, comprising the steps of: (a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes); (b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes); and (c) irradiating the coating of the inner surface of the chamber and the coating of the exterior surface of the sealing member with an isotope, electron beam, or ultraviolet radiation.

In other embodiments, the present invention provides a method for reducing sticktion between an inner surface of a chamber formed from a cyclic polyolefin and an exterior surface of a sealing member of a medical article, comprising the steps of: (a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes); (b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes 500,000 centistokes); and (c) irradiating the coating of the inner surface of the chamber and the coating of the exterior surfaces of the sealing member with an isotope, electron beam, or ultraviolet radiation.

The present invention is more particularly described in the following examples, which are intended to be illustrative only, as numerous modifications and variations therein will be apparent to those skilled in the art.

### EXAMPLE

Syringe barrels for 10 and 50 ml syringes were coated with coating compositions according to the present invention. The syringe barrels were formed from a cyclic polyolefin. The interior surface of each barrel was coated with DC 360 polydimethylsiloxane having a viscosity of 12,500·10⁻⁶ m²/s (12,500 cst), available from Dow Corning. Helvoet FM457 (Butyl-1) and FM460 (Butyl-2) butyl rubber and Kokoku SBR syringe stoppers were coated with a conventional polydimethylsiloxane having a viscosity of 100,000·10⁻⁶ m²/s or 300,000·10⁻⁶ m²/s (100,000 cst or 300,000 cst). The syringe barrels and stoppers were irradiated using gamma radiation at dosages specified in Table 1.

Each syringe was assembled and filled with 10 or 50 ml of deionized water and autoclaved at 124°C for 30 minutes.

Breakout forces, breakloose forces, and sustaining forces may be conveniently measured on a universal mechanical tester or on a testing machine of the type having a constant rate of cross-head movement, as described in detail below. The syringe assemblies were evaluated for breakloose force according to ISO 7886-1 Annex G. The breakloose and sustaining force (in kilograms) of each sample syringe was determined by an Instron Series 5500 at a displacement rate of 100 mm/min according to ISO 7886. The breakloose force is visually determined as the highest peak of the curve or point of where the slope of the curve changes on the graph. The sustaining force is the average force for the stopper to move an additional 45 mm for the 10 ml barrel and 85 mm for the 50 ml barrel after breakloose. The breakloose and sustaining values reported in Table 1 below are the results of four samples for each of Sample Groups 1-10.

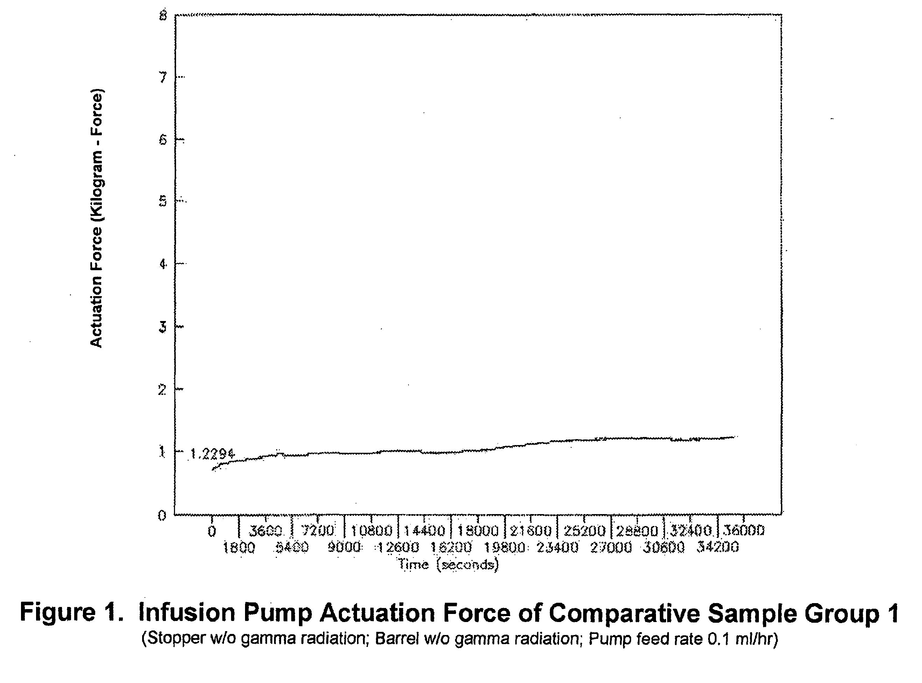

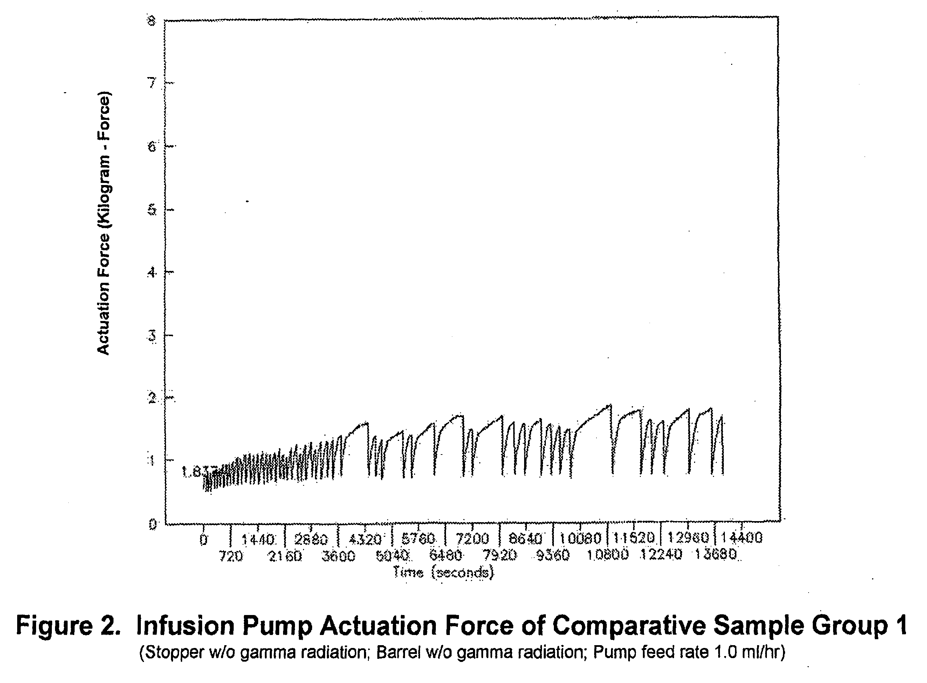

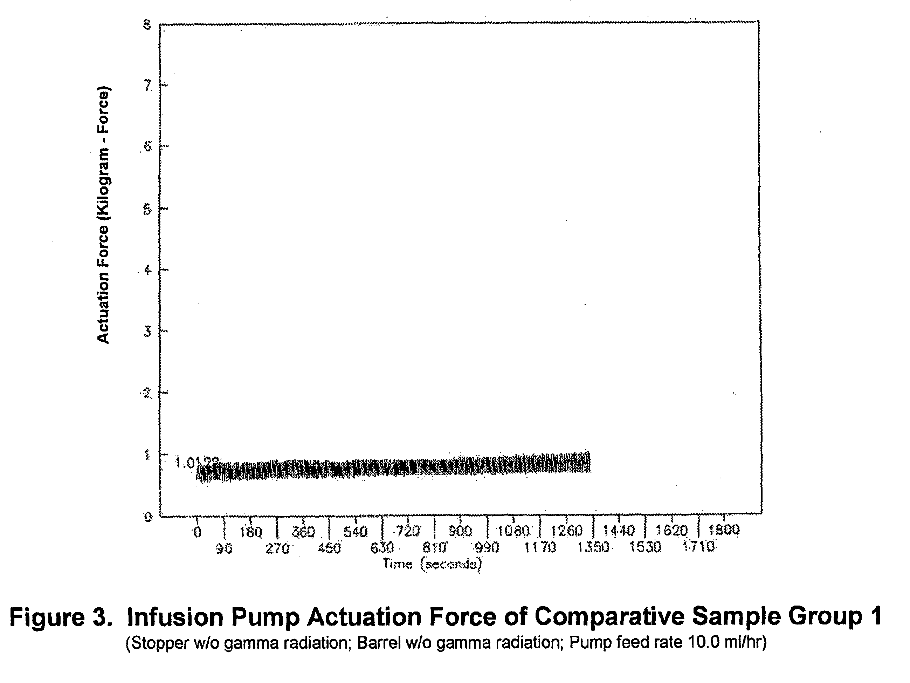

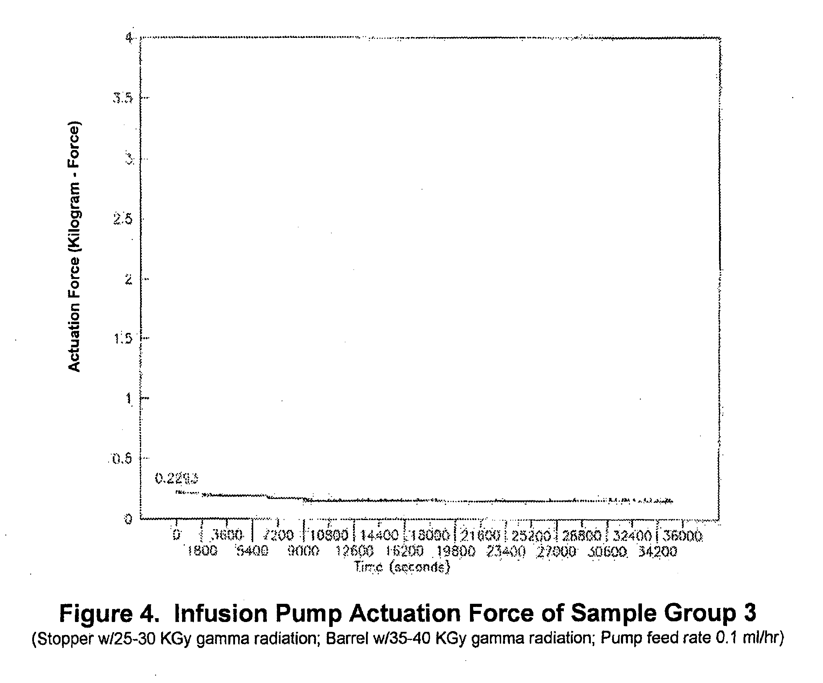

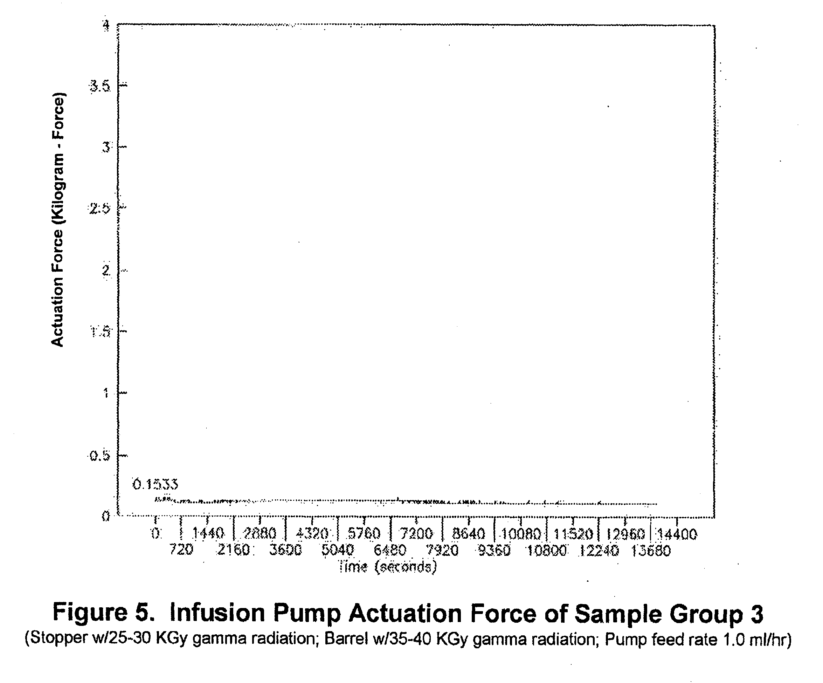

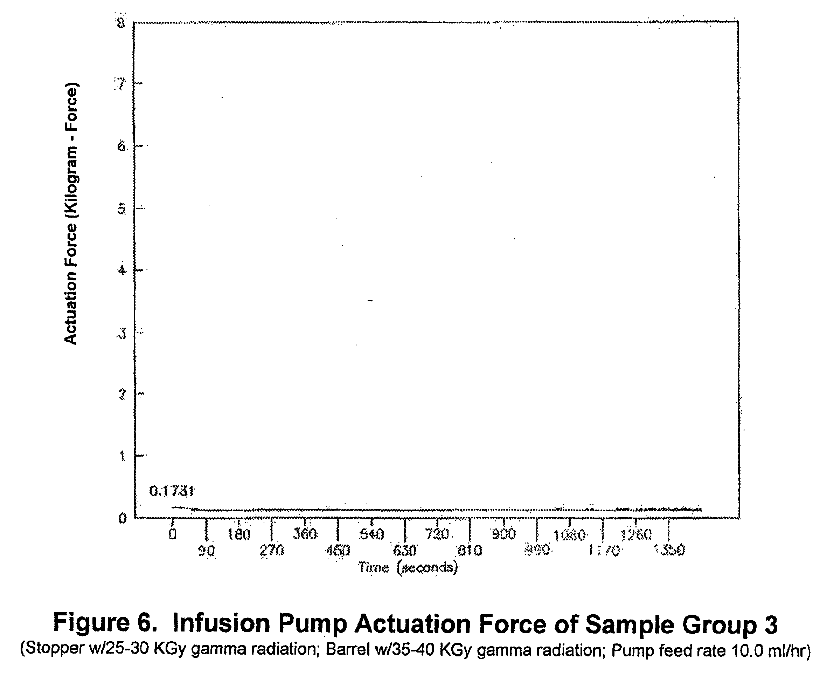

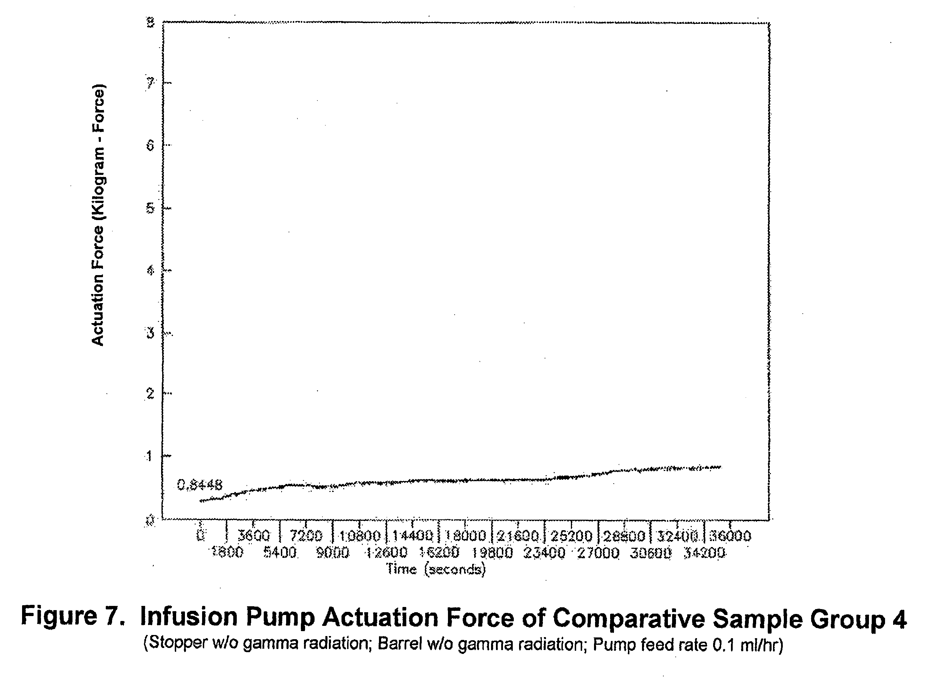

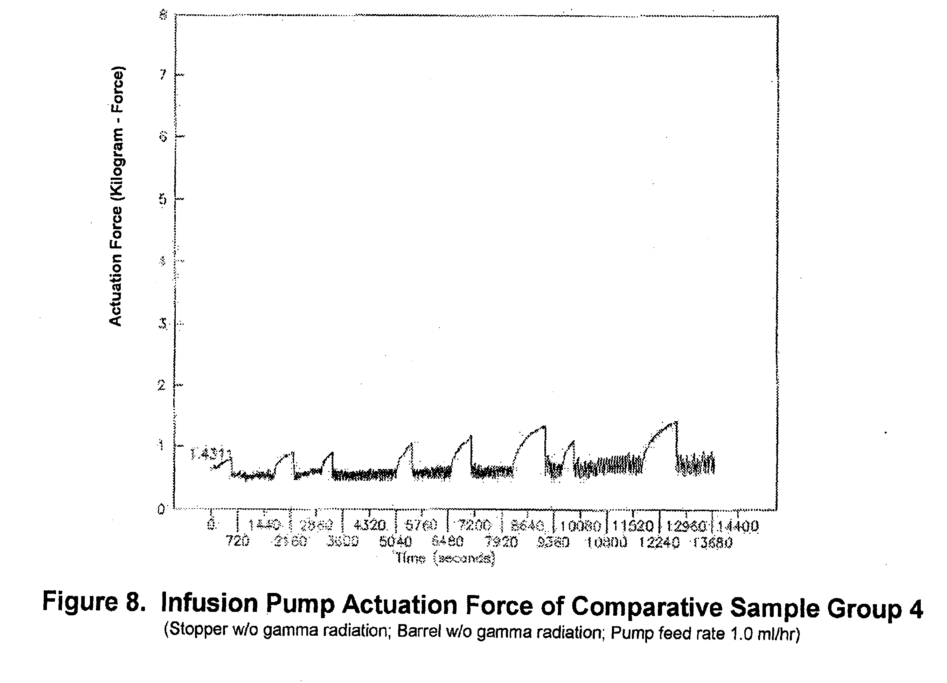

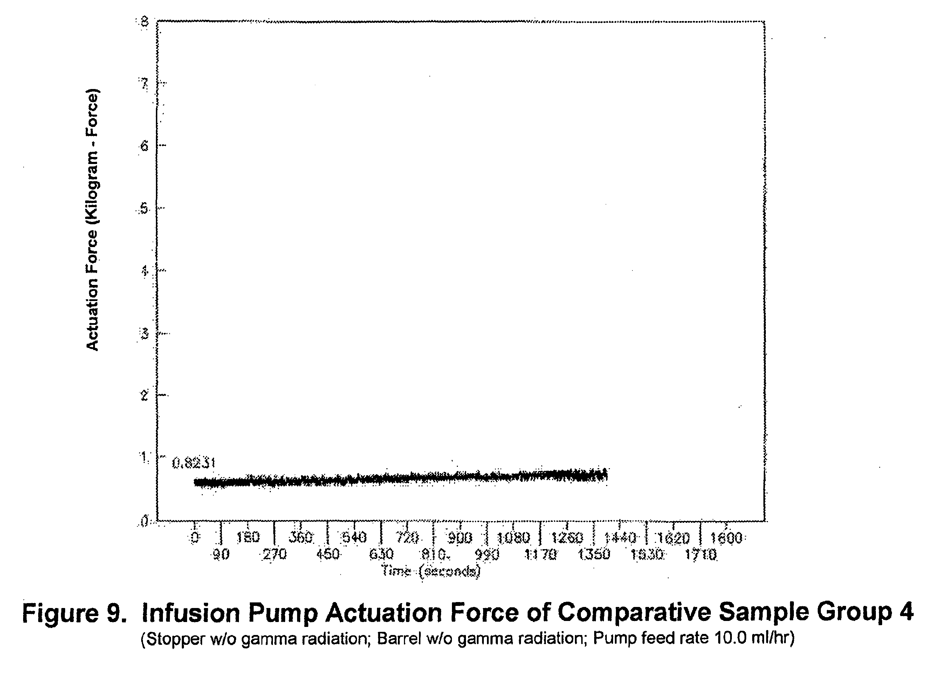

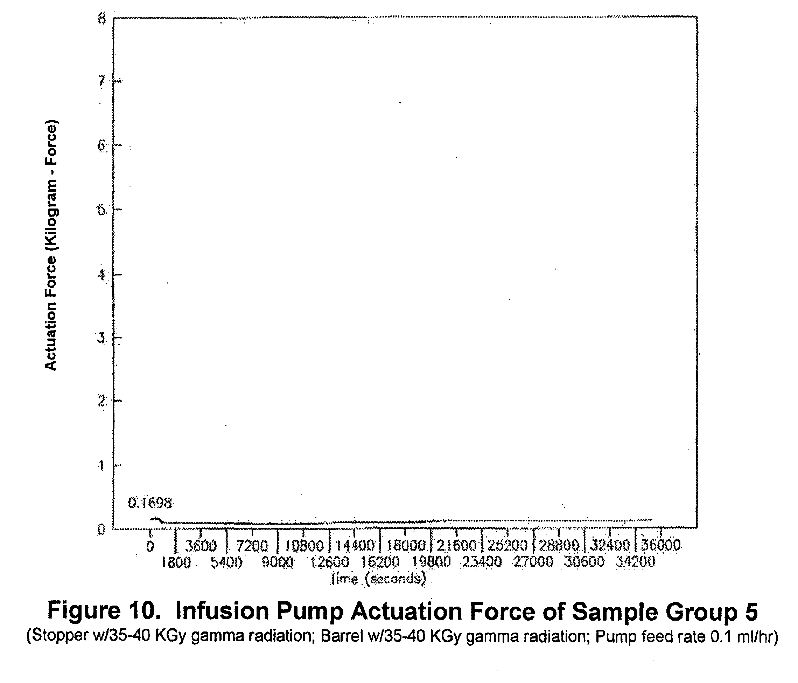

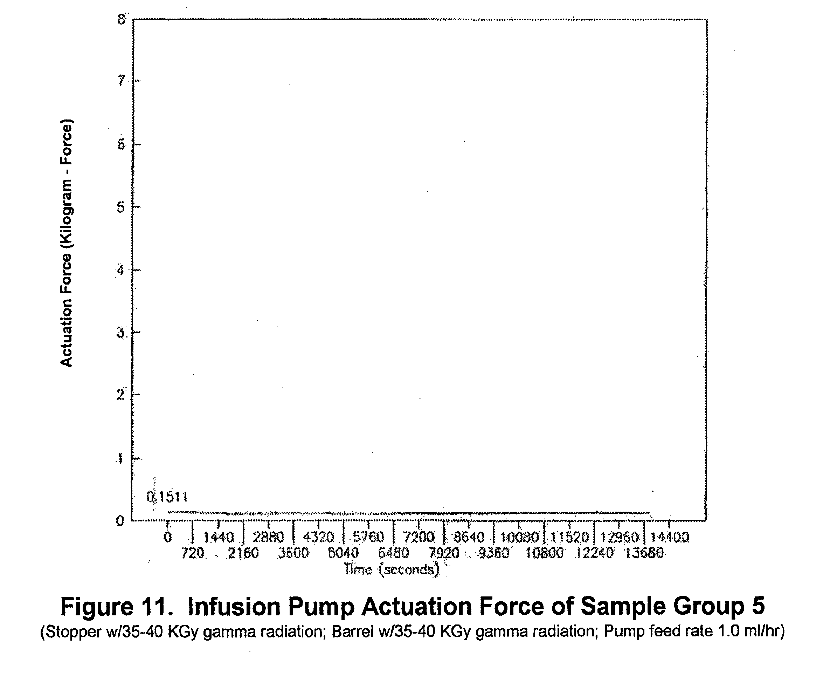

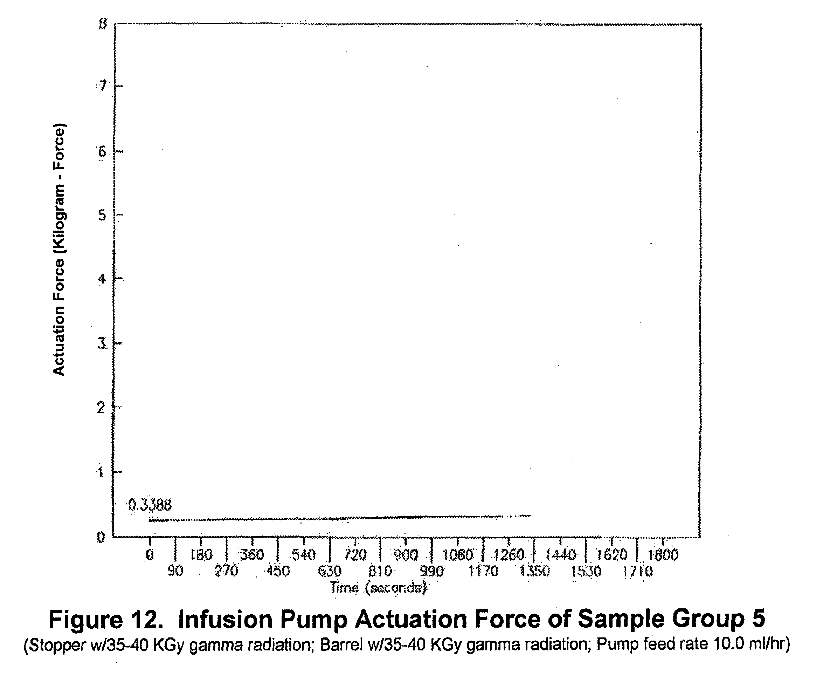

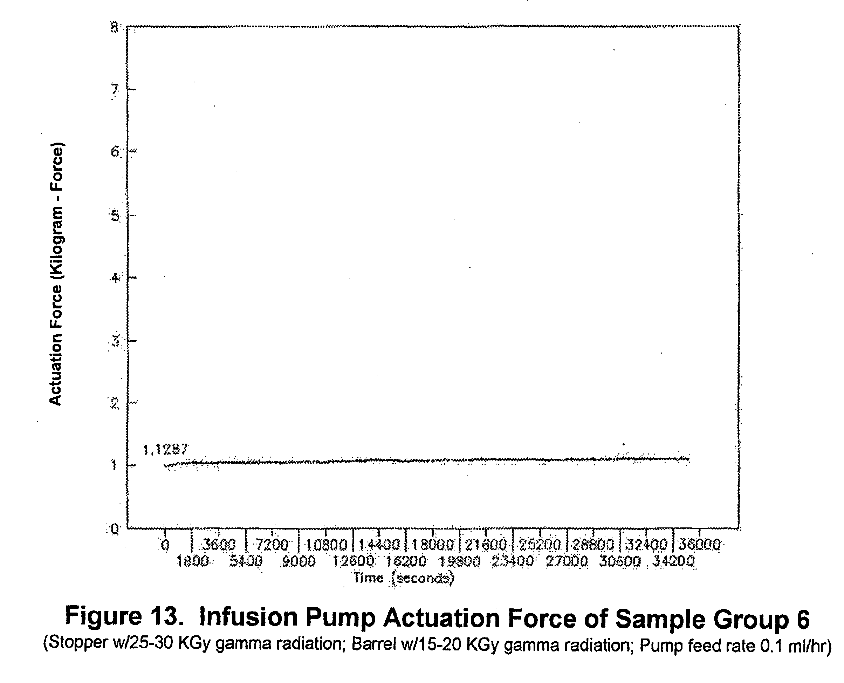

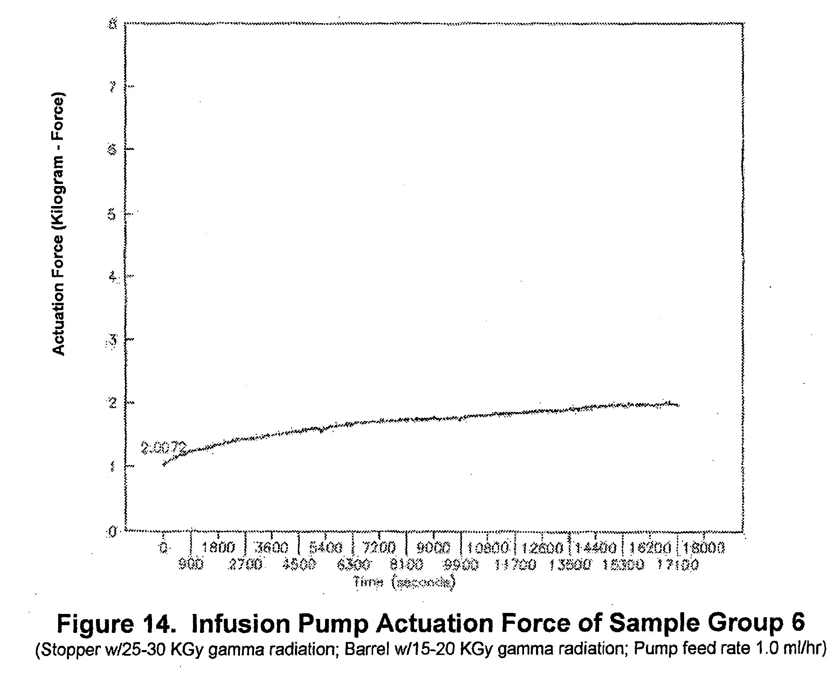

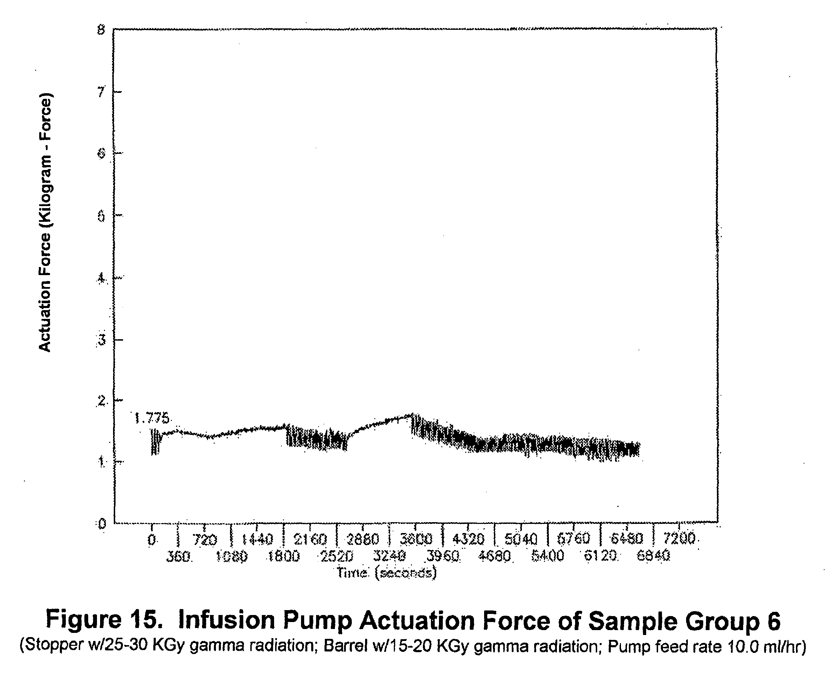

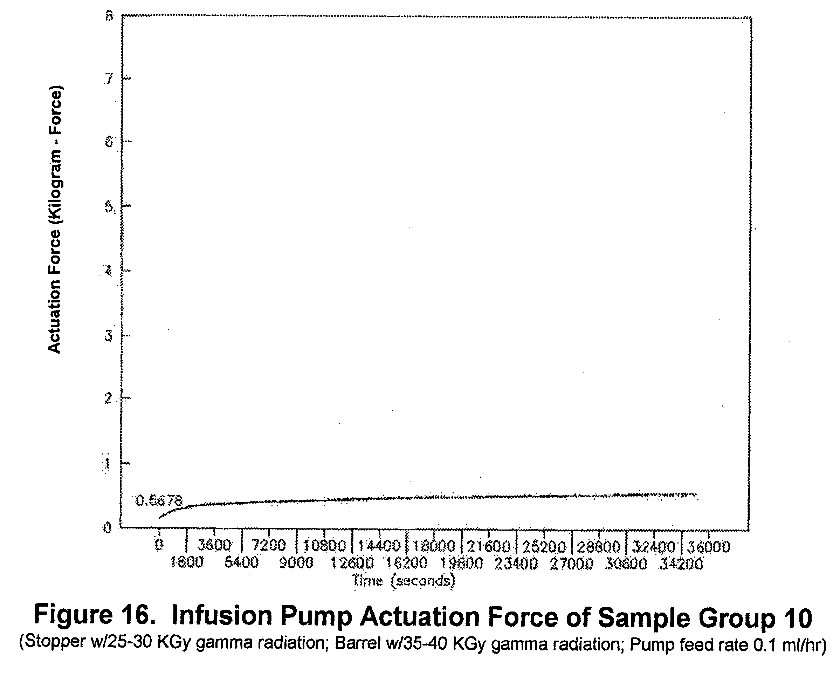

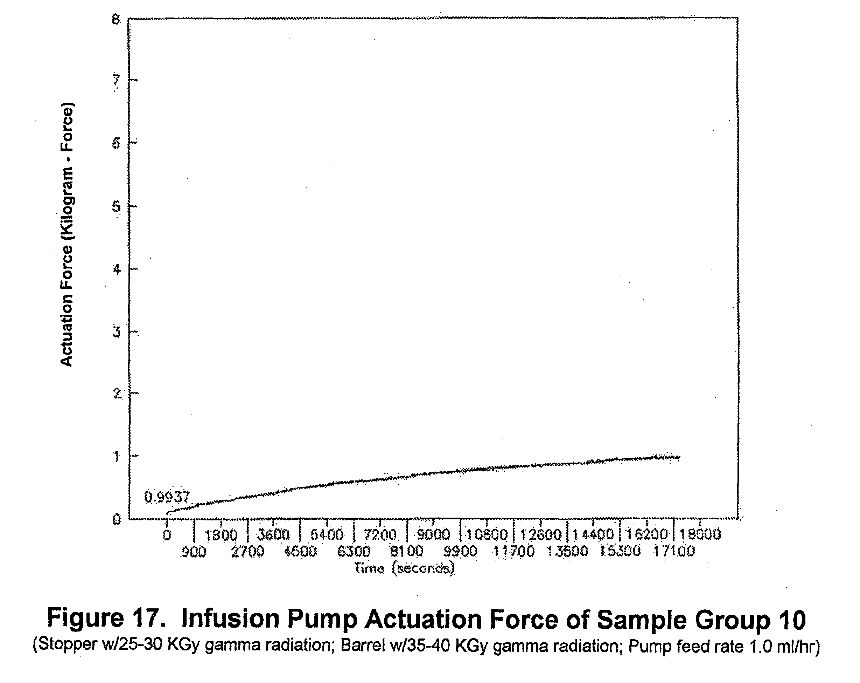

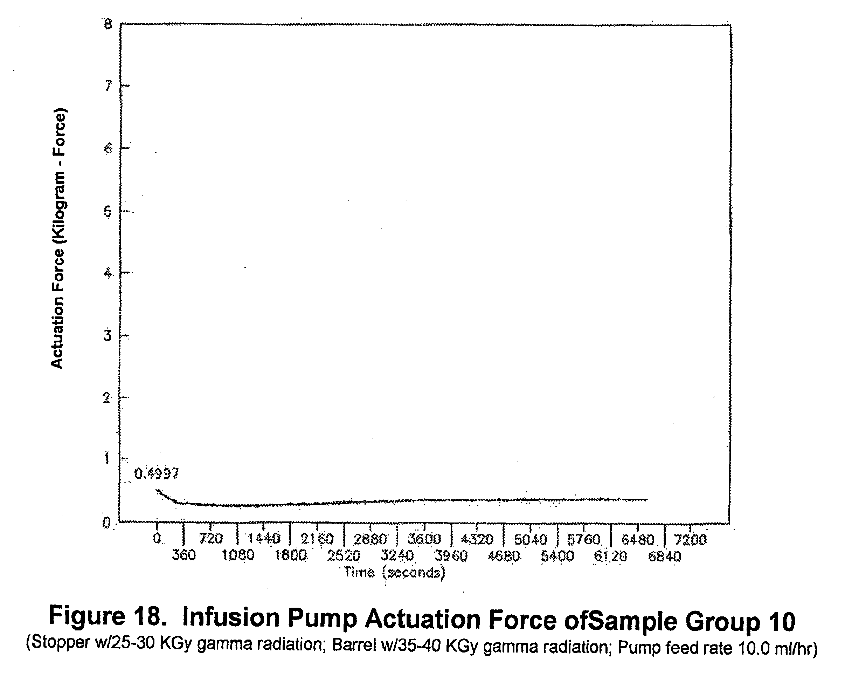

The syringe assemblies were evaluated for infusion pump actuation force according to ISO 7886-2 Annex A. A Becton Dickinson Program 2 syringe pump was used for testing at flow rates of 0.1 ml/hr, 1.0 ml/hr or 10.0 ml/hr. Force was measured using a force transducer placed between the syringe plunger rod and the displacement arm of the pump. A chart of force over time for each syringe was generated, as shown in Figs. 1-18. A visual determination of sticktion or no sticktion was made by viewing each chart for the smoothness of the curve. A smooth curve indicated no sticktion and an irregularly-shaped curve (for example with discernable peaks) indicated sticktion. The actuation force values reported in Table 1 below are the results of four samples for each of Sample Groups 1-10.

As shown in Table 1 below and with reference to Figs. 1-18, the 10 ml Sample Groups 2, 3, and 5 treated with radiation according to the present invention generally exhibited lower breakloose force and reduced sticktion, compared to the Sample Groups 1 and 4, respectively, prepared without radiation treatment. Sample Groups 6-10 treated with radiation according to the present invention generally exhibited no sticktion at 0.1 and 1.0 ml/hr injection rate. Sample Groups 7, 9, and 10, in which the barrel was treated with at least 25 kilogreys of radiation, exhibited lower actuation force, and no sticktion at each speed.

| Table 1. Syringe Sample | 10 ml | | | | | 50 ml | | | | |
|---|---|---|---|---|---|---|---|---|---|---|
| | 1 | 2 | 3 | 4 | 5 | 6 | 7 | 8 | 9 | 10 |
| Stopper | | | | | | | | | | |
| Butyl-1 | x | x | x | | | | | | | |
| SBR | | | | x | x | | | | | |
| Butyl-2 | | | | | | x | x | x | x | x |
| Lube w/100K cst Silicone | x | | | x | x | x | x | | | |
| Lube w/300K cst Silicone | | x | x | | | | | x | x | x |
| No Gamma Irradiation | x | | | x | | | | | | |
| Gamma Irradiation 15-20 KGy | | x | | | | | | | | |
| Gamma Irradiation 25 - 30 KGy | | | x | | | x | x | x | x | x |
| Gamma Irradiation 35 - 40 KGy | | | | | x | | | | | |
| Cyclic Olefin Polymer Barrel | | | | | | | | | | |
| Lube w/12.5K cst Silicone | x | x | x | x | x | x | x | x | x | x |
| No Gamma Irradiation | x | | | x | | | | | | |
| Gamma Irradiation 15 - 20 KGy | | | | | | x | | x | | |
| Gamma Irradiation 25 - 30 KGy | | | | | | | x | | x | |
| Gamma Irradiation 35 - 40 KGy | | x | x | | x | | | | | x |
| Assembly | | | | | | | | | | |
| Fill w DI water & autoclave at 124°C for 30 min | x | x | x | x | x | x | x | x | x | x |
| Actuation Force (Kgf, Boldface w/Sticktion) | | | | | | | | | | |
| At 0.1 ml/h | 1.25 | 0.26 | 0.25 | 0.75 | 0.17 | 0.90 | 0.92 | 1.18 | 0.93 | 0.63 |
| At 1.0 ml/h | 1.86 | 0.18 | 0.13 | 1.17 | 0.17 | 2.01 | 1.69 | 1.95 | 1.74 | 1.20 |
| At 10.0 ml/h | 1.00 | 0.20 | 0.17 | 0.70 | 0.31 | 1.84 | 0.94 | 1.94 | 0.94 | 0.44 |
| Breakloose Force (Kgf) | | | | | | | | | | |
| At 100 mm/min | 7.53 | 1.81 | 1.39 | 2.51 | 0.89 | - | - | - | - | - |
| Sustaining Force (Kgf) | | | | | | | | | | |
| At 100 mm/min | 0.19 | 0.35 | 0.20 | 0.25 | 0.29 | - | - | - | - | - |

The present invention has been described with reference to specific details of particular embodiments thereof. It is not intended that such details be regarded as limitations upon the scope of the invention except insofar as and to the extent that they are included in the accompanying claims.

## Claims

1. A medical article comprising:
(a) a chamber formed from a cyclic polyolefin and having an inner surface in sliding engagement with an exterior surface of a sealing member, wherein the inner surface of the chamber has a coating thereon prepared from a composition comprising a first organopolysiloxane having a viscosity ranging from 5,000·10⁻⁶ m²/s to 100,000·10⁻⁶ m²/s (5,000 centistokes to 100,000 centistokes) as measured with a Brookfield DV II+ viscosimeter, the coating being adhered to the inner surface by crosslinking induced by irradiation with an isotope, electron beam, or ultraviolet radiation; and
(b) a sealing member having an exterior surface in sliding engagement with the interior surface of the chamber, the exterior surface of the sealing member having a coating thereon prepared from a composition comprising a second organopolysiloxane having a viscosity ranging from 10,000·10⁻⁶ m²/s to 500,000·10⁻⁶ m²/s (10,000 centistokes to 500,000 centistokes), the coating being adhered to the exterior surface by crosslinking induced by irradiation with an isotope, electron beam, or ultraviolet radiation, wherein the viscosity of the organopolysiloxane coating the chamber is less than the viscosity of the organopolysiloxane coating the sealing member.

2. The medical article according to claim 1, wherein the medical article is selected from the group consisting of syringe assemblies, drug cartridges, needleless injectors, liquid dispensing devices, and liquid metering devices.

3. The medical article according to claim 1, wherein the chamber is a syringe barrel.

4. The medical article according to claim 1 , wherein at least one of the first organopolysiloxane or the second organopolysiloxane is represented by the following structural formula (I): 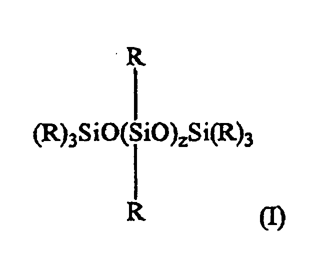 wherein R is alkyl and Z is 30 to 4,500, or
wherein at least one of the first organopolysiloxane or the second organopolysiloxane is polydimethylsiloxane, or
wherein at least one of the first organopolysiloxane or the second organopolysiloxane comprises at least one alkenyl group, preferably
wherein each alkenyl group of the organopolysiloxane is independently selected from the group consisting of vinyl, allyl, propenyl, butenyl, pentenyl, hexenyl, heptenyl, octenyl, nonenyl, and decenyl, or
wherein at least one of the first organopolysiloxane or the second organopolysiloxane comprises at least two polar groups, preferably
wherein each polar group of the organopolysiloxane is independently selected from the group consisting of acrylate, methacrylate, amino, imino, hydroxy, epoxy, ester, alkyloxy, isocyanate, phenolic, polyurethane oligomeric, polyamide oligomeric, polyester oligomeric, polyether oligomeric, polyol, carboxypropyl, and fluoro groups.

5. The medical article according to claim 1, wherein the first organopolysiloxane has a viscosity of 12,500·10⁻⁶ m²/s (12,500 centistokes).

6. The medical article according to claim 1, wherein the second organopolysiloxane has a viscosity of 12,500·10⁻⁶ m²/s to 300,000·10⁻⁶ m²/s (12,500 centistokes to 300,000 centistokes), preferably wherein the second organopolysiloxane has a viscosity of 12,500·10⁻⁶ m²/s (12,500 centistokes), or wherein the second organopolysiloxane has a viscosity of 100,000·10⁻⁶ m²/s (100,000 centistokes), or wherein the second organopolysiloxane has a viscosity of 300,000·10⁻⁶ m²/s (300,000 centistokes).

7. The medical article according to claim 1, wherein the sealing member is selected from the group consisting of a stopper, O-ring, plunger tip and piston.

8. The medical article according to claim 1, wherein the sealing member is formed from rubber, preferably wherein the sealing member is formed from butyl rubber.

9. The medical article according to claim 1, wherein the sealing member is formed from thermoplastic elastomer or thermoplastic vulcanizate, preferably wherein the sealing member is formed from styrene-butadiene copolymer.

10. A method for lubricating the interface between an inner surface of a chamber formed from a cyclic polyolefin and an exterior surface of a sealing member of a medical article, comprising the steps of:
(a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes) as measured with a Brookfield DV II+ viscosimeter;
(b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes),
wherein the viscosity of the organopolysiloxane coating the chamber is less than the viscosity of the organopolysiloxane coating the sealing member; and
(c) irradiating the coating of the inner surface of the chamber and the coating of the exterior surface of the sealing member with an isotope, electron beam, or ultraviolet radiation.

11. The method according to claim 10, wherein the coating of the inner surface of the chamber and the coating of the exterior surface of the sealing member are irradiated concurrently, or wherein the coating of the inner surface of the chamber and the coating of the exterior surface of the sealing member are irradiated consecutively.

12. A method for reducing breakloose force between an inner surface of a chamber formed from a cyclic polyolefin and an exterior surface of a sealing member of a medical article, comprising the steps of:
(a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes) as measured with a Brookfield DV II+ viscosimeter;
(b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes),
wherein the viscosity of the organopolysiloxane coating the chamber is less than the viscosity of the organopolysiloxane coating the sealing member; and
(c) irradiating the coating of the inner surface of the chamber and the coating of the exterior surface of the sealing member with an isotope, electron beam, or ultraviolet radiation.

13. A method for reducing sustaining force between an inner surface of a chamber formed from a cyclic polyolefin and an exterior surface of a sealing member of a medical article, comprising the steps of:
(a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes) as measured with a Brookfield DV II+ viscosimeter;
(b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes),
wherein the viscosity of the organopolysiloxane coating the chamber is less than the viscosity of the organopolysiloxane coating the sealing member; and
(c) irradiating the coating of the inner surface of the chamber and the coating of the exterior surface of the sealing member with an isotope, electron beam, or ultraviolet radiation.

14. A method for reducing sticktion between an inner surface of a chamber formed from a cyclic polyolefin and an exterior surface of a sealing member of a medical article, comprising the steps of:
(a) applying a coating onto an inner surface of the chamber, the coating being prepared from a composition comprising a first organopolysiloxane having a viscosity ranging from 5,000·10⁻⁶ m²/s to 100,000·10⁻⁶ m²/s (5,000 centistokes to 100,000 centistokes) as measured with a Brookfield DV II+ viscosimeter;
(b) applying a coating onto an exterior surface of the sealing member, the coating being prepared from a composition comprising a second organopolysiloxane having a viscosity ranging from 10,000·10⁻⁶ m²/s to 500,000·10⁻⁶ m²/s (10,000 centistokes to 500,000 centistokes),
wherein the viscosity of the organopolysiloxane coating the chamber is less than the viscosity of the organopolysiloxane coating the sealing member; and
(c) irradiating the coating of the inner surface of the chamber and the coating of the exterior surface of the sealing member with an isotope, electron beam, or ultraviolet radiation.

## Patentansprüche

1. Medizinischer Artikel, umfassend:
(a) eine Kammer, die aus einem cyclischen Polyolefin gebildet ist und eine innere Fläche, die gleitend an einer äußeren Fläche eines Dichtungselements angreift, aufweist, wobei die innere Fläche der Kammer eine Beschichtung trägt, die aus einer Zusammensetzung hergestellt ist, welche ein erstes Organopolysiloxan mit einer mittels eines Brookfield-DV-II+-Viskometers gemessenen Viskosität im Bereich von 5000·10⁻⁶ m²/s bis 100 000·10⁻⁶ m²/s (5000 Centistokes bis 100 000 Centistokes) umfasst, wobei die Beschichtung durch Vernetzung, die durch Bestrahlung mit einem Isotop, einem Elektronenstrahl oder ultravioletter Strahlung induziert wird, an der inneren Fläche haftend gemacht wird; und
(b) ein Dichtungselement, das eine äußere Fläche, die gleitend an der inneren Fläche der Kammer angreift, aufweist, wobei die äußere Fläche des Dichtungselements eine Beschichtung trägt, die aus einer Zusammensetzung hergestellt ist, welche ein zweites Organopolysiloxan mit einer Viskosität im Bereich von 10 000·10⁻⁶ m²/s bis 500 000·10⁻⁶ m²/s (10 000 Centistokes bis 500 000 Centistokes) umfasst, wobei die Beschichtung durch Vernetzung, die durch Bestrahlung mit einem Isotop, einem Elektronenstrahl oder ultravioletter Strahlung induziert wird, an der äußeren Fläche haftend gemacht wird, wobei die Viskosität des Organopolysiloxans, mit dem die Kammer beschichtet ist, kleiner ist als die Viskosität des Organopolysiloxans, mit dem das Dichtungselement beschichtet ist.

2. Medizinischer Artikel gemäß Anspruch 1, wobei der medizinische Artikel aus der Gruppe ausgewählt ist, die aus Spritzenvorrichtungen, Medikamentenpatronen, nadellosen Injektoren, Flüssigkeitsverabreichungsvorrichtungen und Flüssigkeitsdosiervorrichtungen besteht.

3. Medizinischer Artikel gemäß Anspruch 1, wobei die Kammer ein Spritzenzylinder ist.

4. Medizinischer Artikel gemäß Anspruch 1, wobei das erste Organopolysiloxan und/oder das zweite Organopolysiloxan durch die folgende Strukturformel (I) dargestellt wird: 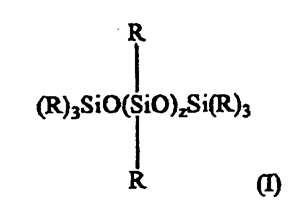 wobei R = Alkyl ist und z = 30 bis 4500 beträgt; oder
wobei es sich bei dem ersten Organopolysiloxan und/oder bei dem zweiten Organopolysiloxan um Polydimethylsiloxan handelt; oder
wobei das erste Organopolysiloxan und/oder das zweite Organopolysiloxan wenigstens eine Alkenylgruppe umfasst, wobei vorzugsweise
jede Alkenylgruppe des Organopolysiloxans unabhängig aus der Gruppe ausgewählt ist, die aus Vinyl, Allyl, Propenyl, Butenyl, Pentenyl, Hexenyl, Heptenyl, Octenyl, Nonenyl und Decenyl besteht; oder
wobei das erste Organopolysiloxan und/oder das zweite Organopolysiloxan wenigstens zwei polare Gruppen umfasst, wobei vorzugsweise jede polare Gruppe des Organopolysiloxans unabhängig aus der Gruppe ausgewählt ist, die aus Acrylat-, Methacrylat-, Amino-, Imino-, Hydroxy-, Epoxy-, Ester-, Alkyloxy-, Isocyanat-, Phenol-, Polyurethanoligomer-, Polyamidoligomer-, Polyesteroligomer-, Polyetheroligomer-, Polyol-, Carboxypropyl- und Fluorgruppen besteht.

5. Medizinischer Artikel gemäß Anspruch 1, wobei das erste Organopolysiloxan eine Viskosität von 12 500·10⁻⁶ m²/s (12 500 Centistokes) hat.

6. Medizinischer Artikel gemäß Anspruch 1, wobei das zweite Organopolysiloxan eine Viskosität von12 500·10⁻⁶ m²/s bis 300 000·10⁻⁶ m²/s (12 500 Centistokes bis 300 000 Centistokes) hat, wobei vorzugsweise das zweite Organopolysiloxan eine Viskosität von 12 500·10⁻⁶ m²/s (12 500 Centistokes) hat oder wobei das zweite Organopolysiloxan eine Viskosität von 100 000·10⁻⁶ m²/s (100 000 Centistokes) hat oder wobei das zweite Organopolysiloxan eine Viskosität von 300 000·10⁻⁶ m²/s (300000 Centistokes) hat.

7. Medizinischer Artikel gemäß Anspruch 1, wobei das Dichtungselement aus der Gruppe ausgewählt ist, die aus einem Stopfen, O-Ring, einem Tauchkolbenvorderteil und einem Kolben besteht.

8. Medizinischer Artikel gemäß Anspruch 1, wobei das Dichtungselement aus Gummi besteht, wobei das Dichtungselement vorzugsweise aus Butylkautschuk besteht.

9. Medizinischer Artikel gemäß Anspruch 1, wobei das Dichtungselement aus einem thermoplastischen Elastomer oder thermoplastischen Vulkanisat besteht, wobei das Dichtungselement vorzugsweise aus Styrol-Butadien-Copolymer besteht.

10. Verfahren zum Schmieren der Grenzfläche zwischen einer inneren Fläche einer Kammer, die aus einem cyclischen Polyolefin gebildet ist, und einer äußeren Fläche eines Dichtungselements eines medizinischen Artikels, umfassend die Schritte:
(a) Auftragen einer Beschichtung auf eine innere Fläche der Kammer, wobei die Beschichtung aus einer Zusammensetzung hergestellt ist, die ein erstes Organopolysiloxan mit einer mittels eines Brookfield-DV-II+-Viskometers gemessenen Viskosität im Bereich von 5000·10⁻⁶ m²/s bis 100 000·10⁻⁶ m²/s (5000 Centistokes bis 100 000 Centistokes) umfasst;
(b) Auftragen einer Beschichtung auf eine äußere Fläche des Dichtungselements, wobei die Beschichtung aus einer Zusammensetzung hergestellt ist, die ein zweites Organopolysiloxan mit einer Viskosität im Bereich von 10 000·10⁻⁶ m²/s bis 500 000·10⁻⁶ m²/s (10 000 Centistokes bis 500 000 Centistokes) umfasst,
wobei die Viskosität des Organopolysiloxans, mit dem die Kammer beschichtet ist, kleiner ist als die Viskosität des Organopolysiloxans, mit dem das Dichtungselement beschichtet ist; und
(c) Bestrahlen der Beschichtung der inneren Fläche der Kammer und der Beschichtung der äußeren Fläche des Dichtungselements mit einem Isotop, einem Elektronenstrahl oder ultravioletter Strahlung.

11. Verfahren gemäß Anspruch 10, wobei die Beschichtung der inneren Fläche der Kammer und die Beschichtung der äußeren Fläche des Dichtungselements gleichzeitig bestrahlt werden oder wobei die Beschichtung der inneren Fläche der Kammer und die Beschichtung der äußeren Fläche des Dichtungselements nacheinander bestrahlt werden.

12. Verfahren zum Reduzieren der Loslösekraft zwischen einer inneren Fläche einer Kammer, die aus einem cyclischen Polyolefin gebildet ist, und einer äußeren Fläche eines Dichtungselements eines medizinischen Artikels, umfassend die Schritte:
(a) Auftragen einer Beschichtung auf eine innere Fläche der Kammer, wobei die Beschichtung aus einer Zusammensetzung hergestellt ist, die ein erstes Organopolysiloxan mit einer mittels eines Brookfield-DV-II+-Viskometers gemessenen Viskosität im Bereich von 5000·10⁻⁶ m²/s bis 100 000·10⁻⁶ m²/s (5000 Centistokes bis 100 000 Centistokes) umfasst;
(b) Auftragen einer Beschichtung auf eine äußere Fläche des Dichtungselements, wobei die Beschichtung aus einer Zusammensetzung hergestellt ist, die ein zweites Organopolysiloxan mit einer Viskosität im Bereich von 10 000·10⁻⁶ m²/s bis 500 000·10⁻⁶ m²/s (10 000 Centistokes bis 500 000 Centistokes) umfasst,
wobei die Viskosität des Organopolysiloxans, mit dem die Kammer beschichtet ist, kleiner ist als die Viskosität des Organopolysiloxans, mit dem das Dichtungselement beschichtet ist; und
(c) Bestrahlen der Beschichtung der inneren Fläche der Kammer und der Beschichtung der äußeren Fläche des Dichtungselements mit einem Isotop, einem Elektronenstrahl oder ultravioletter Strahlung.

13. Verfahren zum Reduzieren der Weitergleitkraft zwischen einer inneren Fläche einer Kammer, die aus einem cyclischen Polyolefin gebildet ist, und einer äußeren Fläche eines Dichtungselements eines medizinischen Artikels, umfassend die Schritte:
(a) Auftragen einer Beschichtung auf eine innere Fläche der Kammer, wobei die Beschichtung aus einer Zusammensetzung hergestellt ist, die ein erstes Organopolysiloxan mit einer mittels eines Brookfield-DV-II+-Viskometers gemessenen Viskosität im Bereich von 5000·10⁻⁶ m²/s bis 100 000·10⁻6 m²/s (5000 Centistokes bis 100 000 Centistokes) umfasst;
(b) Auftragen einer Beschichtung auf eine äußere Fläche des Dichtungselements, wobei die Beschichtung aus einer Zusammensetzung hergestellt ist, die ein zweites Organopolysiloxan mit einer Viskosität im Bereich von 10 000·10⁻⁶ m²/s bis 500 000·10⁻⁶ m²/s (10 000 Centistokes bis 500 000 Centistokes) umfasst,
wobei die Viskosität des Organopolysiloxans, mit dem die Kammer beschichtet ist, kleiner ist als die Viskosität des Organopolysiloxans, mit dem das Dichtungselement beschichtet ist; und
(c) Bestrahlen der Beschichtung der inneren Fläche der Kammer und der Beschichtung der äußeren Fläche des Dichtungselements mit einem Isotop, einem Elektronenstrahl oder ultravioletter Strahlung.

14. Verfahren zum Reduzieren der Haftreibung zwischen einer inneren Fläche einer Kammer, die aus einem cyclischen Polyolefin gebildet ist, und einer äußeren Fläche eines Dichtungselements eines medizinischen Artikels, umfassend die Schritte:
(a) Auftragen einer Beschichtung auf eine innere Fläche der Kammer, wobei die Beschichtung aus einer Zusammensetzung hergestellt ist, die ein erstes Organopolysiloxan mit einer mittels eines Brookfield-DV-II+-Viskometers gemessenen Viskosität im Bereich von 5000·10⁻⁶ m²/s bis 100 000·10⁻⁶ m²/s (5000 Centistokes bis 100 000 Centistokes) umfasst;
(b) Auftragen einer Beschichtung auf eine äußere Fläche des Dichtungselements, wobei die Beschichtung aus einer Zusammensetzung hergestellt ist, die ein zweites Organopolysiloxan mit einer Viskosität im Bereich von 10 000·10⁻⁶ m²/s bis 500 000·10⁻⁶ m²/s (10 000 Centistokes bis 500 000 Centistokes) umfasst,
wobei die Viskosität des Organopolysiloxans, mit dem die Kammer beschichtet ist, kleiner ist als die Viskosität des Organopolysiloxans, mit dem das Dichtungselement beschichtet ist; und
(c) Bestrahlen der Beschichtung der inneren Fläche der Kammer und der Beschichtung der äußeren Fläche des Dichtungselements mit einem Isotop, einem Elektronenstrahl oder ultravioletter Strahlung.

## Revendications

1. Article médical comprenant :
(a) une chambre formée à partir d'une polyoléfine cyclique et ayant une surface intérieure coulissant par contact avec une surface extérieure d'un élément faisant étanchéité, la surface intérieure de la chambre étant recouverte d'un revêtement préparé à partir d'une composition comprenant un premier organopolysiloxane ayant une viscosité dans la plage allant de 5 000 * 10-⁶ m²/s à 100 000 * 10⁻⁶ m²/s (5 000 centistokes à 100 000 centistokes) telle que mesurée par un viscosimètre Brookfield DV II+, le revêtement adhérant à la surface intérieure par réticulation induite par irradiation par un isotope, un faisceau d'électrons, ou un rayonnement ultraviolet ; et
(b) un élément faisant étanchéité ayant une surface extérieure coulissant par contact avec la surface intérieure de la chambre, la surface extérieure de l'élément faisant étanchéité étant recouverte d'un revêtement préparé à partir d'une composition comprenant un second organopolysiloxane ayant une viscosité dans la plage allant de 10 000 * 10⁻⁶ m²/s à 500 000 * 10⁻⁶ m²/s (10 000 centistokes à 500 000 centistokes), le revêtement adhérant à la surface extérieure par réticulation induite par irradiation par un isotope, un faisceau d'électrons, ou un rayonnement ultraviolet, la viscosité du revêtement en organopolysiloxane de la chambre étant inférieure à la viscosité du revêtement en organopolysiloxane de l'élément faisant étanchéité.

2. Article médical selon la revendication 1, l'article médical étant choisi dans le groupe comprenant des ensembles de seringues, des cartouches de médicaments, des injecteurs sans aiguilles, des dispositifs d'administration de liquides, et des dispositifs de mesure de liquides.

3. Article médical selon la revendication 1, dans lequel la chambre est un cylindre de seringue.

4. Article médical selon la revendication 1, dans lequel au moins un parmi le premier organopolysiloxane ou le second organopolysiloxane est représenté par la formule de structure (I) suivante : 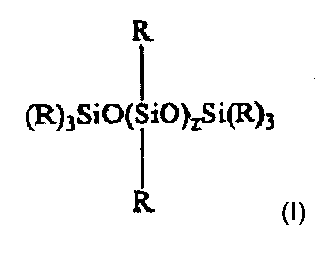 dans laquelle R représente un alkyle et Z représente un nombre allant de 30 à 4 500, ou
dans laquelle au moins un parmi le premier organopolysiloxane ou le second organopolysiloxane est un polydiméthylsiloxane, ou
dans laquelle au moins un parmi le premier organopolysiloxane ou le second organopolysiloxane comprend au moins un groupe alcényle, de préférence
chaque groupe alcényle des organopolysiloxanes étant indépendamment choisi dans le groupe consistant en les vinyle, allyle, propényle, butényle, pentényle, hexényle, heptényle, octényle, nonényle, et décényle, ou
dans lequel au moins un parmi le premier organopolysiloxane ou le second organopolysiloxane comprend au moins deux groupes polaires, de préférence
chaque groupe polaire des organopolysiloxanes étant indépendamment choisi dans le groupe consistant en les acrylate, méthacrylate, amino, imino, hydroxy, époxy, ester, alkyloxy, isocyanate, les composés phénoliques, les oligomères polyuréthanes, les oligomères polyamides, les oligomères polyesters, les oligomères polyéthers, les polyols, les groupes carboxypropyle, et les groupes fluoro.

5. Article médical selon la revendication 1, dans lequel le premier organopolysiloxane a une viscosité de 12 500 * 10⁻⁶ m²/s (12 500 centistokes).

6. Article médical selon la revendication 1, dans lequel le second organopolysiloxane a une viscosité allant de 12 500 * 10⁻⁶ m²/s à 300 000 * 10⁻⁶ m²/s (12 500 centistokes à 300 000 centistokes), de préférence dans lequel le second organopolysiloxane a une viscosité de 12 500 * 10⁻⁶ m²/s (12 500 centistokes), ou dans lequel le second organopolysiloxane a une viscosité de 100 000 * 10⁻⁶ m²/s (100 000 centistokes), ou dans lequel le second organopolysiloxane a une viscosité de 300 000 * 10⁻⁶ m²/s (300 000 centistokes).

7. Article médical selon la revendication 1, dans lequel l'élément faisant étanchéité est choisi dans le groupe consistant en un bouchon, un joint torique, une pointe de piston et un piston.

8. Article médical selon la revendication 1, dans lequel l'élément faisant étanchéité est formé à partir de caoutchouc, de préférence dans lequel l'élément faisant étanchéité est formé à partir de caoutchouc butylique.

9. Article médical selon la revendication 1, dans lequel l'élément faisant étanchéité est formé à partir d'élastomère thermoplastique ou de vulcanisat thermoplastique, de préférence dans lequel l'élément faisant étanchéité est formé à partir de copolymère styrène-butadiène.

10. Procédé destiné à lubrifier l'interface entre une surface intérieure d'une chambre formée à partir d'une polyoléfine cyclique et une surface extérieure d'un élément faisant étanchéité d'un article médical, comprenant les étapes consistant à :
(a) appliquer un revêtement sur une surface intérieure de la chambre, le revêtement étant préparé à partir d'une composition comprenant un premier organopolysiloxane ayant une viscosité dans la plage allant de 5 000 * 10⁻⁶ m²/s à 100 000 * 10⁻⁶ m²/s (5 000 centistokes à 100 000 centistokes) telle que mesurée par un viscosimètre Brookfield DV II+ ;
(b) appliquer un revêtement sur une surface extérieure de l'élément faisant étanchéité, le revêtement étant préparé à partir d'une composition comprenant un second organopolysiloxane ayant une viscosité dans la plage allant de 10 000 * 10⁻⁶ m²/s à 500 000 * 10⁻⁶ m²/s (10 000 centistokes à 500 000 centistokes),
la viscosité du revêtement en organopolysiloxane de la chambre étant inférieure à la viscosité du revêtement en organopolysiloxane de l'élément faisant étanchéité ; et
(c) irradier le revêtement de la surface intérieure de la chambre et le revêtement de la surface extérieure de l'élément faisant étanchéité avec un isotope, un faisceau d'électrons, ou un rayonnement ultraviolet.

11. Procédé selon la revendication 10, dans lequel le revêtement de la surface intérieure de la chambre et le revêtement de la surface extérieure de l'élément faisant étanchéité sont irradiés simultanément, ou dans lequel le revêtement de la surface intérieure de la chambre et le revêtement de la surface extérieure de l'élément faisant étanchéité sont irradiés consécutivement.

12. Procédé destiné à réduire la force de décollement entre une surface intérieure d'une chambre formée à partir d'une polyoléfine cyclique et une surface extérieure d'un élément faisant étanchéité d'un article médical, comprenant les étapes consistant à :
(a) appliquer un revêtement sur une surface intérieure de la chambre, le revêtement étant préparé à partir d'une composition comprenant un premier organopolysiloxane ayant une viscosité dans la plage allant de 5 000 * 10⁻⁶ m²/s à 100 000 * 10⁻⁶ m²/s (5 000 centistokes à 100 000 centistokes) telle que mesurée par un viscosimètre Brookfield DV II+ ;
(b) appliquer un revêtement sur une surface extérieure de l'élément faisant étanchéité, le revêtement étant préparé à partir d'une composition comprenant un second organopolysiloxane ayant une viscosité dans la plage allant de 10 000 * 10⁻⁶ m²/s à 500 000 * 10⁻⁶ m²/s (10 000 centistokes à 500 000 centistokes),
la viscosité du revêtement en organopolysiloxane de la chambre étant inférieure à la viscosité du revêtement en organopolysiloxane de l'élément faisant étanchéité ; et
(c) irradier le revêtement de la surface intérieure de la chambre et le revêtement de la surface extérieure de l'élément faisant étanchéité avec un isotope, un faisceau d'électrons, ou un rayonnement ultraviolet.

13. Procédé destiné à réduire la force de frottement entre une surface intérieure d'une chambre formée à partir d'une polyoléfine cyclique et une surface extérieure d'un élément faisant étanchéité d'un article médical, comprenant les étapes consistant à :
(a) appliquer un revêtement sur une surface intérieure de la chambre, le revêtement étant préparé à partir d'une composition comprenant un premier organopolysiloxane ayant une viscosité dans la plage allant de 5 000 * 10⁻⁶ m²/s à 100 000 * 10⁻⁶ m²/s (5 000 centistokes à 100 000 centistokes) telle que mesurée par un viscosimètre Brookfield DV II+ ;
(b) appliquer un revêtement sur une surface extérieure de l'élément faisant étanchéité, le revêtement étant préparé à partir d'une composition comprenant un second organopolysiloxane ayant une viscosité dans la plage allant de 10 000 * 10⁻⁶ m²/s à 500 000 * 10⁻⁶ m²/s (10 000 centistokes à 500 000 centistokes),
la viscosité du revêtement en organopolysiloxane de la chambre étant inférieure à la viscosité du revêtement en organopolysiloxane de l'élément faisant étanchéité ; et
(c) irradier le revêtement de la surface intérieure de la chambre et le revêtement de la surface extérieure de l'élément faisant étanchéité avec un isotope, un faisceau d'électrons, ou un rayonnement ultraviolet.

14. Procédé destiné à réduire l'adhérence entre une surface intérieure d'une chambre formée à partir d'une polyoléfine cyclique et une surface extérieure d'un élément faisant étanchéité d'un article médical, comprenant les étapes consistant à :
(a) appliquer un revêtement sur une surface intérieure de la chambre, le revêtement étant préparé à partir d'une composition comprenant un premier organopolysiloxane ayant une viscosité dans la plage allant de 5 000 * 10⁻⁶ m²/s à 100 000 * 10⁻⁶ m²/s (5 000 centistokes à 100 000 centistokes) telle que mesurée par un viscosimètre Brookfield DV II+ ;
(b) appliquer un revêtement sur une surface extérieure de l'élément faisant étanchéité, le revêtement étant préparé à partir d'une composition comprenant un second organopolysiloxane ayant une viscosité dans la plage allant de 10 000 * 10⁻⁶ m²/s à 500 000 * 10⁻⁶ m²/s (10 000 centistokes à 500 000 centistokes),
la viscosité du revêtement en organopolysiloxane de la chambre étant inférieure à la viscosité du revêtement en organopolysiloxane de l'élément faisant étanchéité ; et
(c) irradier le revêtement de la surface intérieure de la chambre et le revêtement de la surface extérieure de l'élément faisant étanchéité avec un isotope, un faisceau d'électrons, ou un rayonnement ultraviolet.
